# EUROPÄISCHE PATENTSCHRIFT

(11) **EP 2 311 009 B1**
(45) Veröffentlichungstag und Bekanntmachung des Hinweises auf die Patenterteilung: **08.02.2012**
(21) Anmeldenummer: 09777057.2
(22) Anmeldetag: 08.07.2009
(51) Int. Cl.: G06T 19/00

(54) **VERFAHREN, SYSTEM, VORRICHTUNG UND COMPUTERPROGRAMM ZUR ERSTELLUNG EINES PROTHESENSCHAFTES**
METHOD, SYSTEM, APPARATUS AND COMPUTER PROGRAM FOR CREATING A PROSTHESIS SOCKET
PROCÉDÉ, SYSTÈME, DISPOSITIF ET PROGRAMME INFORMATIQUE POUR CRÉER UNE EMBOÎTURE DE PROTHÈSE

(30) Priorität: 14.07.2008 DE 102008032992
(43) Veröffentlichungstag der Anmeldung: 20.04.2011
(73) Patentinhaber: Pro Thesis GmbH, 86154 Augsburg (DE)
(72) Erfinder: SCHOTTDORF, Bernd, 89561 Dischingen (DE)
(74) Vertreter: Stammberger, Tobias
(86) Internationale Anmeldenummer: PCT/EP2009/004968
(87) Internationale Veröffentlichungsnummer: WO 2010/006728

(56) Entgegenhaltungen:
- EP-A- 1 843 291
- GIORGIO COLOMBO ET AL: "ICT Methodologies to Model and Simulate Parts of Human Body for Prosthesis Design" DIGITAL HUMAN MODELING; [LECTURE NOTES IN COMPUTER SCIENCE], SPRINGER BERLIN HEIDELBERG, BERLIN, HEIDELBERG, Bd. 4561, 22. Juli 2007 (2007-07-22), Seiten 559-568, XP019063787 ISBN: 978-3-540-73318-8
- TANIA DOUGLAS ET AL: "Ultrasound Imaging in Lower Limb Prosthetics" IEEE TRANSACTIONS ON NEURAL SYSTEMS AND REHABILITATIONENGINEERING, IEEE SERVICE CENTER, NEW YORK, NY, US, Bd. 10, Nr. 1, 1. März 2002 (2002-03-01), XP011078071 ISSN: 1534-4320
- COLOMBO G., BERTETTI M., BONACINI D., MAGRASSI G.: "Reverse Engineering and Rapid Prototyping Techniques to Innovate Prosthesis Socket Design" SPIE, PO BOX 10 BELLINGHAM WA 98227-0010 USA, Bd. 6056, 27. Januar 2006 (2006-01-27), Seiten P1-P11, XP040217446

## Beschreibung

### GEBIET DER ERFINDUNG

Die vorliegende Erfindung betrifft ein Verfahren, ein System, eine Vorrichtung und ein Computerprogramm zur Erzeugung und Modifizierung eines 3D-Schaft-Stumpfmodells zur Herstellung eines Prothesenschaftes zur Verbindung eines einen Stumpf bildenden Körperteils mit einer Prothese.

### HINTERGRUND DER ERFINDUNG

Bisher werden Prothesen für amputierte Patienten, insbesondere Unterschenkel- und Oberschenkelprothesen, auf handwerkliche Art und Weise hergestellt, indem ein Orthopädietechniker ein Gipsabdrucknegativ von einem Stumpf des Patienten abformt. Der Orthopädiefachmann und/oder der Orthopädietechniker ermittelt durch manuelles Abtasten (Palpieren) des Stumpfes zusätzlich anatomische Information, die in eine manuelle Nachbearbeitung eines vom Gipsnegativ erstellten Stumpfpositivmodells einfließt. Das vom Orthopädietechniker nachbearbeitete Stumpfpositivmodell dient als Ausgangsbasis für die Herstellung eines steifen oder flexiblen Schafts, je nach verwendetem Material, z.B. Thermoplaste, Gießharze und Holz. Zur visuellen Kontrolle von Druckstellen wird meist ein thermoplastischer Klarsichtschaft über das Stumpfpositivmodell tiefgezogen. Dauerhafte Schäfte aus Gießharz verstärkt der Orthopädiemechaniker mit Einlegegeflechten, z.B. Karbon-Kevlar-Geflecht, Kohlefasern oder Glasfasergeflecht. Das Ziel war es bisher einen möglichst optimal am Stumpf anliegenden Schaft herzustellen, der am Stumpf übermäßigen Druck und Reibung verhindert, jedoch auch guten Halt und Tragekomfort bietet. Dieses Ziel wird jedoch regelmäßig nicht erreicht, so dass mehrere im Volumen unterschiedliche so genannte Probeschäfte, z.B. ein thermoplastischer Klarsichtschaft, herzustellen sind bevor ein dauerhafter so genannter Definitivschaft für den Patienten beschwerdefrei einsetzbar ist.

Die Dissertation von M. Hasenpusch mit dem Titel "Beitrag zur Optimierung der Stumpfdatenerfassung und computergestützten Stumpfbettgestaltung" hatte zur Aufgabe einen Schaft ausgehend von einem belasteten und unbelasteten Patientenstumpf unter Einsatz rechnergestützter Modellierung herzustellen. Hasenpusch beschreibt den Einsatz von CAD/CAM-Systemen zur rechnergestützten Prothesenschaftkonstruktion (CASD, engl. Computer Aided Socket Design).

Die Dissertation Hasenpusch stellt unter anderem das so genannten San Antonio-System der Universität of Texas San Antonio vor. Das System arbeitet zur Herstellung von Unterschenkelstumpfbettungen mit einem Digitizer, Laserscanner, Ultraschall, Computertomographie (CT) und Magnetresonanztomographie (MRT). Digital erfasste Daten des Stumpfes wandelt das San Antonio System in eine topografische Oberfläche um und stellt diese als Gittermodell dar. Das System kann ein vollständiges 3D-Bild oder Querschichten darstellen. Verarbeitet das San Antonio-System CT-Bilder, dann sind nur Modifikationen an Querschnittskonturen möglich. Erfolgt die Verarbeitung von Laser- und Digitizerdaten, ermöglicht das System Querschnitts-, Profilmodellierung und Modifikationen am 3D-Gittermodell. Die Fertigung des Schafts erfolgt mittels einer 3-Achsen-CNC-Fräsmaschine.

Ferner nimmt die Dissertation Hasenpusch Bezug auf die Miterfassung der ossären Struktur durch CT, MRT und Ultraschall, für den Fall, dass der Orthopädietechniker manuell ein Modell am Rechner anpasst ohne zuvor einen Gipsabdruck angefertigt zu haben. Hasenpusch beschreibt außerdem als Forschungsziel mit CT und MRT als bildgebende Verfahren eine geometrische Beschreibung sowohl der äußeren als auch der knöchernen Kontur eines Stumpfes zu gewinnen.

Die Offenlegungsschrift DE 10 2005 008 605 der Fa. Gottinger Orthopädie-Technik GmbH beschreibt ein Verfahren zur Herstellung einer äußeren Prothese oder Orthese mit den Schritten: Erstellen einer Schichtaufnahme des betreffenden Körperteils; Umwandeln der erstellten Daten in ein 3D-Modell; Auswerten der Knochen-, Muskelund Fettgewebestruktur; Ermitteln von Kompressionszonen in Abhängigkeit von der Auswertung und Erstellen eines Prothesen-/Orthesenelements in Abhängigkeit von diesen Daten.

Die DE 10 2005 008 605 der Fa. Gottinger Orthopädie-Technik GmbH beschreibt die Verwendung von CT- oder MRT-Aufnahmen eines Oberschenkelstumpfes sowie einer CAD-Software zur Erstellung eines 3D-Modells und ggf. dessen Modulation von zuvor generierten Zweckformen. Bei dem 3D-Modell sind Muskulatur und Fettgewebe ersichtlich. Außerdem kann die Formgebung eines virtuell erstellten Prothesenschaftes am Rechner optimiert werden.

Eine weitere Druckschrift der der Fa. Gottinger Orthopädie-Technik GmbH, die EP 1 843 291 A1, beschreibt ein objektiviertes Verfahren zur Erstellung eines Prothesenschaftes für einen Extremitätenstumpf eines Patienten, welches bei der Erstellung eines stetigen dreidimensionalen Modells Informationen über die Lage von Muskel-, Fett- und Knochengewebe im Stumpf berücksichtigt, eine Ziel-Kompression auf Grundlage des Gewichtes des Patienten und der Außenfläche des Stumpfes bestimmt und welches die Kompressibilität der Anteile von Muskel- und Fettgewebe berücksichtigt.

Die wissenschaftliche Veröffentlichung "ICT Methodologies to Model and Simulate Parts of Human Body for Prothesis Design", G. Colombo et al., Digital Human Modeling, Lecture Notes in Computer Science, 4561, 559-568, 22. Juli 2007 beschreibt ein Verfahren zur Simulation von menschlichen Körperteilen, um virtuell einen passgenauen Prothesenschaft für einen Patienten zu entwerfen. Dazu verbindet die beschriebene Lehre hochentwickelte Reverse Engeneering Methoden, Simulations- und Modellierungsverfahren und die Techniken des Rapid Prototyping. Aufgabe der vorliegenden Erfindung ist es, ein verbessertes und insbesondere die tatsächliche Gewebestruktur des Stumpfes besser berücksichtigendes Verfahren, ein System, Vorrichtung und Computerprogramm zur Erstellung eines Prothesenschaftes bereitzustellen, mit dem optimal an den Stumpf angepasste Prothesenschäfte herstellbar sind.

### ZUSAMMENFASSUNG DER ERFINDUNG

Erfindungsgemäß wird diese Aufgabe durch ein Verfahren, eine Vorrichtung, ein System und ein Computerprogramm nach den unabhängigen Ansprüchen gelöst. Varianten und bevorzugte Ausführungsformen der Erfindung ergeben sich aus den abhängigen Ansprüchen, der nachfolgenden Beschreibung und den Zeichnungen.

Ein Aspekt der Erfindung betrifft ein Verfahren zur Erstellung eines 3D-Schaft-Stumpfmodells zur Herstellung eines Prothesenschaftes zur Verbindung eines einen Stumpf bildenden Körperteils mit einer Prothese die folgenden Schritte. Als erster Schritt werden dreidimensionale (3D) Bilddaten des den Stumpf bildenden Körperteils, das mehrere Gewebearten, wie Haut, Fett, Muskeln und Knochen umfasst, akquiriert. Vorteilhafterweise liefert eine Computertomographie (CT) oder Magnetresonanztomographie (MRT) hochauflösende Schichtaufnahmen des den Stumpf bildenden Körperteils bei vorbestimmtem Schichtabstand und vorbestimmter Schichtdicke, wobei mehrere nacheinander akquirierte Schichtaufnahmen 3D-Bilddaten in Form von Volumendaten bilden können. Der Einsatz von Magnetresonanztomographie (MRT) statt der CT hat den Vorteil geringerer (Röntgen-)Strahlenbelastung für den Patienten. Zur Ergänzung von CT und/oder MRT können herkömmliche Röntgen-, Sonographieaufnahmen, optoelektronische Aufnahmen oder andere bildgebende Verfahren verwendet werden. Die Kombination von CT und/oder MRT und/oder Röntgen und/oder Sonographie liefert vorteilhafterweise genauere 3D-Bilddaten über die innere und äußere Beschaffenheit des Stumpfes.

Ein Speichermedium, wie z.B. CD/DVD, Festplatte, USB-Stick oder dergleichen kann die akquirierten 3D-Bilddaten speichern, die in einem vorbestimmten Format vorliegen. Vorteile eines solchen Speichermediums liegen in einer schnellen Archivier- und Transportierbarkeit der 3D-Bilddaten. Außerdem können die 3D-Bilddaten an einem ersten entfernten Ort akquiriert werden, der in räumlicher Distanz von einem zweiten zentralen Ort liegt, an dem nachfolgende Verarbeitungsschritte der 3D-Bilddaten durchgeführt werden können.

Ferner kann ein Transfer der 3D-Bilddaten von dem ersten Ort zum zweiten Ort durchgerührt werden, z.B. via Internet oder dergleichen. Der Vorteil liegt dabei in einer räumlichen Trennung des ersten Ortes, an dem beispielsweise ein CT-Gerät die 3D-Bilddaten akquiriert, und dem zweiten Ort, an dem weitere Verarbeitungsschritte durchgeführt werden können.

Vorzugsweise wird der DICOM-Standard (Digital Imaging and Communications in Medicine) verwendet. Der Standard kann sowohl ein erstes Speicherformat der 3D-Bilddaten festlegen als auch deren Kommunizierung.

Vorzugsweise wird nach dem Speichern und/oder Übertragen der 3D-Bilddaten ein Konvertierverfahren auf die 3D-Bilddaten angewendet. Die resultierenden 3D-Bilddaten können in einem zweiten Speicherformat gespeichert und/oder weiter übertragen werden. Das Konvertierverfahren kann beispielsweise von einer Konvertiereinheit durchgeführt werden, die sich am zweiten zentralen Ort befindet. Die Konvertiereinheit umfasst vorzugsweise einen Server, Prozessor, DSP-Chip oder dergleichen. Das andere Speicherformat umfasst z.B. JPEG, GIF, TIFF, BMP, PDF oder dergleichen.

Die 3D-Bilddaten, welche Informationen über die Stumpfbeschaffenheit beinhalten, werden vorzugsweise in einem weiteren Schritt des Verfahrens einer Segmentierung unterzogen, die der Bestimmung der Verteilung von Gewebearten des Stumpfes dient. Die Segmentierung liefert eine Zuordnung der Bildpunkte zu verschiedenen Gewebearten und damit eine Unterscheidung der Gewebearten, wobei die Gewebearten Haut, Fett, Muskeln und Knochen, etc. umfassen. Vorzugsweise kann die Segmentierung durch Schwellwerte, beispielsweise Grauwerte, genauer in weitere Gewebearten unterteilen, wobei die Gewebearten eine zu bestimmende räumliche Verteilung aufweisen. Den Gewebearten können Eigenschaften, wie z.B. Kompressibilität, Dichte, Festigkeit, Schmerzempfinden und/oder Schmerzsensibilität etc. zugeordnet werden.

Die segmentierten 3D-Bilddaten werden vorzugsweise in einem weiteren Schritt des Verfahrens zu einem 3D-Schaft-Stumpfmodell rekonstruiert. Diese Rekonstruktion ist ein Vorgang, der aufeinanderfolgende zweidimensionale Schichtaufnahmen der segmentierten 3D-Bilddaten des Stumpfes beispielsweise durch Interpolation in ein 3D-Schaft-Stumpfmodell umwandelt. Das 3D-Schaft-Stumpfmodell beschreibt die Geometrie des Stumpfes und die Verteilung wenigstens einer segmentierten Gewebeart des Stumpfes. Auch das tatsächliche Volumen des Stumpfes wird durch das 3D-Schaft-Stumpfmodell wieder gegeben. Somit entspricht das 3D-Schaft-Stumpfmodell einer genauen Widergabe des Stumpfes, wenn man einmal von möglichen Fehlern beim bildgebenden Verfahren wie beispielsweise Messungenauigkeiten, Interpolations- und Rundungsfehlern absieht.

Vorteilhafterweise sind die vorgenannten Vorgänge Segmentieren der 3D-Bilddaten und Rekonstruieren des 3D-Schaft-Stumpfmodells, die an den ersten Schritt Akquirieren von 3D-Bilddaten anschließen, automatisch, ohne den Patienten und ohne einen Orthopädietechniker durchführbar.

In einem weiteren Schritt des Verfahrens wird wenigstens eine Stumpfachse im 3D-Schaft-Stumpfmodell festgelegt. Beispielsweise kann dies manuell durch einen Benutzer oder vollautomatisch erfolgen. Vorzugsweise wird die räumliche Lage der Stumpfachse durch einen oberen und einen unteren Stumpfachsenpunkt festgelegt. Beispielsweise führt die Stumpfachse entlang einer Kraftwirkungsrichtung, die am Stumpf bildenden Körperteil auftreten kann. So kann die Stumpfachse längs des 3D-Schaft-Stumpfmodells durch den Femur (Oberschenkelknochen) verlaufen oder ist durch das Articulatio coxae (Hüftgelenk) und das Articulatio genus (Kniegelenk) gemäß der Biomechanik und/oder Motorik festlegbar. Alternativ kann der obere Stumpfachsenpunkt vorzugsweise an der Fossa acetabuli liegen. Der untere Stumpfachsenpunkt kann alternativ an einem distalen Bereich des Stumpfes durch geometrische Mittelung definiert werden. Orthogonal zur Stumpfachse können zwei weitere Achsen festgelegt werden, um vorzugsweise dreidimensionale Raumrichtungen und Maßangaben definieren zu können.

Das Verfahren umfasst weiter die Schritte: Unterteilen wenigstens eines Bereiches des 3D-Schaft-Stumpfmodells in wenigstens eine Scheibe bestimmter Dicke bzw. eine Schicht bestimmten Abstandes im Wesentlichen senkrecht zur Stumpfachse und Unterteilen der wenigstens einen Scheibe bzw. Schicht in Winkelsektoren. Alternativ kann die wenigstens eine Scheibe bzw. Schicht im Wesentlichen senkrecht zum Femur im 3D-Schaft-Stumpfmodell bestimmt werden. Dabei wird die wenigstens eine Scheibe in eine vorbestimmte Anzahl von Winkelsektoren gleichen und/oder verschiedenen Winkelanteils unterteilt. Die Winkelsektoren bilden in der Scheibe einen Vollkreis, wobei deren Winkelanteile in der Summe 360° betragen. Vorzugsweise wird die wenigstens eine Scheibe in wenigstens zwei Winkelsektoren unterteilt. Beispielsweise kann ein 3D-Schaft-Stumpfmodell umso passgenauer erstellt werden, je höher die Anzahl an Winkelsektoren ist.

In einem weiteren Schritt des Verfahrens erfolgt ein Modifizieren des 3D-Schaft-Stumpfmodells auf der Grundlage von wissenbasierten Regelsätzen zur optimalen Anpassung des 3D-Schaft-Stumpfmodells an den Stumpf, wobei die wissensbasierten Regelsätze auf wenigstens einen Winkelsektor der wenigstens einen Scheibe bzw. Schicht angewendet werden. Die wissensbasierten Regelsätze berücksichtigen einerseits die in dem 3D-Schaft-Stumpfmodell enthaltenen Informationen über Geometrie des Stumpfes und/oder Verteilung wenigstens einer Gewebeart. Andererseits umfassen wissensbasierte Regeln ein oder mehrere Regeln, die eine oder mehrere Eigenschaften der wenigstens einen segmentierten Gewebeart verwenden. Eigenschaften der wenigstens einen segmentierten Gewebeart sind vorzugsweise physikalische Größen und physiologische Eigenschaften, beispielsweise Dichte, Kompressibilität, Festigkeit und Schmerzempfinden oder ein Wert bzw. Faktor, der die Volumenkompression angibt.

Die Festlegung einer Stumpfachse kann automatisch ebenfalls anhand der wissensbasierten Regelsätze erfolgen. Vorzugsweise wird das Verfahren zur Erstellung eines 3D-Schaft-Stumpfmodells zur Herstellung eines Prothesenschaftes zur Verbindung eines einen Stumpf bildenden Körperteils mit einer Prothese bereitgestellt.

Während des Akquirierens der 3D-Bilddaten des den Stumpf bildenden Körperteils ist am Stumpf vorzugsweise ein sog. Liner angebracht. Dieser gibt eine gewünschte Form des Stumpfes bei der Bildaufnahme vor, um beispielsweise einer künstlichen Querformung des Stumpfes entgegenzuwirken. Die gewünschte Form entspricht dabei idealerweise der Form des Stumpfes in dem Zustand, in dem der Patient aufrecht steht, in welcher die Schwerkraft entlang der Körperlängsachse verläuft. Ferner kann die Wahl eines Materials, das der Liner umfasst, für die Segmentierung geeignet sein, d.h. das Material hat vorteilhafterweise eine hohe oder niedrige Dichte, um Gewebearten des Stumpfes von umliegenden Gewebearten, die nicht zum Stumpf gehören, in den 3D-Bilddaten unterscheiden zu können. Der Liner umfasst vorzugsweise Silikon, Polyurethan (PU) oder ähnliches Material.

Das Akquirieren von 3D-Bilddaten kann bei einem Patienten, der auf seiner dem Stumpf gegenüberliegenden Körperseite liegt, erfolgen, wobei sein dem Stumpf gegenüberliegendes Bein angewinkelt wird. Das Anwinkeln bewirkt vorteilhafterweise eine entspannte Beckenposition und eine korrekte Beugestellung des Stumpfes.

Gemäß einem weiteren Ausführungsbeispiel umfasst das Segmentieren der 3D-Bilddaten ein Segmentieren anhand von 2D-Darstellungen der 3D-Bilddaten. Die 2D-Darstellungen können zweidimensionale Schnittbilder sein, welche den Schichtaufnahmen des bildgebenden Verfahrens entsprechen oder ähnlichen, anhand der zweidimensionalen Schichtaufnahmen durch ein Interpolationsverfahren ermittelten Schnittbildern. Vorzugsweise können die 2D-Darstellungen Schnittbilder sein, welche senkrecht zu einer anhand medizinischer Kriterien definierten Stumpfachse angeordnet sind.

Nach einem weiteren Ausführungsbeispiel umfasst das Segmentieren der 3D-Bilddaten eine Umrisserkennung und/oder eine Vektorisierung. Vorzugsweise werden bei der Umrisserkennung einzelne Schichtbilder aus den 3D-Daten anhand ihrer Farbwerte analysiert. Da verschiedene Gewebearten jeweils ähnliche Farbwerte aufweisen, können sie dadurch vorzugsweise voneinander abgegrenzt und/oder unterschieden werden. Die Vektorisierung von 3D-Bilddaten erzeugt aus getrennten Bildpunkten eine Geometrie, die mathematisch definierte Flächen und/oder Kurven aufweist.

Vorzugsweise werden zum Rekonstruieren eines 3D-Schaft-Stumpfmodells segmentierte, d.h. vektorisierte und mit Umrissen versehene 3D-Bilddaten verwendet. Das Rekonstruieren liefert ein 3D-Schaft-Stumpfmodell, das ein Stumpf bildendes Körperteil nachbildet. Durch ein geeignetes Darstellungsverfahren oder ein Darstellungsprogramm für das rekonstruierte 3D-Schaft-Stumpfmodell kann bspw. eine Vorderansicht des Modells oder eine Aufsicht auf verschiedene zweidimensionale Schnittbilder durch das 3D-Schaft-Stumpfmodell dem Benutzer angezeigt werden. Anhand der Modelldarstellung kann durch den Orthopädietechniker oder den Arzt bevorzugt eine Fehlstellung des Stumpfes ermittelt werden. Dazu kann ein Fehlstellungsdreieck anhand anatomisch eindeutig aus den zweidimensionalen Schnittbildern ableitbarer Strukturen (bspw. Knochenstrukturen im Beckenbereich) konstruiert werden, welches ein Maß einer Außenrotation des Stumpfes zur Körperachse des Patienten anzeigt. Die Ermittlung eines Fehlstellungsdreieckes kann bevorzugt automatisch erfolgen.

Eine Rekonstruktionseinheit zum Rekonstruieren kann am zweiten zentralen Ort angeordnet ausgelegt sein, um das 3D-Schaftstumpfmodel zu rekonstruieren und/oder zu speichern und/oder weiter zu übertragen. Das Speichern des 3D-Schaft-Stumpfmodells kann in einem dritten vorbestimmten Speicherformat erfolgen. Das 3D-Schaft-Stumpfmodell kann vorteilhafterweise Gewebearten und Größeninformationen des den Stumpf bildenden Körperteils umfassen.

Die wissensbasierten Regelsätze modifizieren das 3D-Schaft-Stumpfmodell vorzugsweise so, dass das Modifizieren in einer verbesserten Kraftübertragung zwischen Stumpf und Prothesenschaft resultiert.

Die Anwendung wissensbasierter Regelsätze zum Modifizieren eines 3D-Schaft-Stumpfmodells umfasst dabei vorzugsweise Erfahrungswerte, z.B. aus Messreihen von Schaft-Probanden ermittelte empirische Werte zu physikalischen, physiologischen und anatomischen Eigenschaften bestimmter Gewebebereiche, in Form wenigstens einer mathematischen Transformation. Das Modifizieren des 3D-Schaft-Stumpfmodells anhand solcher wissensbasierter Regelsätze führt vorzugsweise zu Volumen- oder Formänderung des 3D-Schaft-Stumpfmodells.

Die wissensbasierten Regelsätze können wenigstens eine der folgenden Gewebeeigenschaften für wenigstens eine der verschiedenen Gewebearten als Gewebeparameter umfassen: Schmerzempfinden, Festigkeit, Dichte und Kompressibilität oder einen Wert bzw. Faktor, der die Volumenkompression angibt.

Weiter können die wissensbasierten Regelsätze auch wenigstens einen der Parameter Körpergewicht des Patienten und Stumpf-Außenfläche umfassen, wobei die Stumpf-Außenfläche anhand des 3D-Schaft-Stumpfmodells automatisch durch Flächenberechnung ermittelt werden kann. Um den Tragekomfort des zu erstellenden Schaftes weiter zu erhöhen, kann es von Vorteil sein, anhand der Parameter Körpergewicht und Stumpf-Außenfläche eine durch die Modifikation des 3D-Schaft-Stumpfmodells zu erzielende Schaft-Modifikation zu bestimmen. Bevorzugt handelt es sich bei der Modifikation um eine Volumenänderung oder Kompression. Eine zu erzielende Volumenkompression kann mittels eines Proportionalitätsfaktors, beispielsweise der Kompressibilität einer bestimmten Gewebeart oder mehrerer Gewebearten, in eine Druckverteilung über das gesamte 3D-Schaft-Stumpfmodell überführt werden. Bei der Kompressibilität handelt es sich um die physikalische Größe κ, welche eine relative Volumenänderung infolge einer Druckänderung beschreibt. Besonders vorteilhaft kann sich bei der Ermittlung einer Druckverteilung auswirken, neben den Kompressibilitäten der verschiedenen Gewebearten auch die Lage von Knochenstrukturen oder den Beginn des Femurs im distalen Bereich des 3D-Schaft-Stumpfmodells zu berücksichtigen. So wird beispielsweise bevorzugt keine Modifikation des 3D-Schaft-Stumpfmodells unterhalb des Femurs im distalen Bereich vorgenommen.

Nach einer weiteren Ausführungsform der Erfindung hängen die vorab festgelegten Winkelsektor-Unterteilungen, aus denen der Benutzer für die Scheibe auswählen kann, von der Lage der Scheibe bzw. Schicht innerhalb des Stumpfes, der Gewebeverteilung in der Scheibe und/oder physiologischen und anatomischen Eigenschaften des Stumpfes bzw. des Patienten ab. Die Festlegung oder eine Anpassung der vorab festgelegten räumlichen Verteilungen erfolgt bevorzugt durch den Benutzer oder automatisch. Für eine automatische Anpassung durchlaufen die wissensbasierten Regelsätze bevorzugt einen Selbstlernprozess. Es ist von Vorteil, wenn sich die wissensbasierten Regeln vollständig oder teilweise durch Anwenden bestimmter Selbstlernverfahren oder dergleichen automatisieren lassen. Vorzugsweise lernen wissensbasierte Regeln selbst, indem in einem oder mehreren Stümpfen von verschiedenen Patienten enthaltene Informationen über die Geometrie und/oder Verteilung der wenigstens einen segmentierten Gewebeart gespeichert und analysiert werden. Die wissensbasierten Regeln können beispielsweise auswerten, welche Winkelsektor-Unterteilung besonders häufig beim Modifizieren eines 3D-Schaft-Stumpfmodells in einem bestimmten Abschnitt verwendet wurde und eine Ordnung der Winkelsektor-Unterteilungen nach deren Wichtigkeit vornehmen. Entsprechend dieser Ordnung können einem Benutzer Vorschläge für Winkelsektor-Einteilungen gemacht werden. Darüber hinaus kann sich das Selbstlernen auch auf die Zuordnung der Kompressionsfaktoren zu den einzelnen Winkelsektoren beziehen. Der meist verwendete Kompressionsfaktor kann dem Benutzer zusammen mit einer Winkelsektor-Unterteilung bevorzugt zur Auswahl vorgeschlagen werden. Der Selbstlernprozess erfolgt immer unter Berücksichtigung der in einem Bereich des 3D-Schaft-Stumpfmodells vorliegenden Gewebeverteilung. Ferner können die wissensbasierten Regelsätze auch Eingaben des Benutzers, bspw. eine Auswahl eines zu verwendenden Kompressionsfaktors, bei ihrem Selbstlernprozess auswerten. So kann von Patient zu Patient, dessen 3D-Schaft-Stumpfmodell mit dem beschriebenen Verfahren modifiziert wird, eine Optimierung des Verfahrensablaufes und verbesserte Anpassung des Schafts an den Stumpf des Patienten erzielt werden.

Nach einer weiteren Ausführungsform der Erfindung berücksichtigen die vorab festgelegten Winkelsektor-Unterteilungen, aus denen der Benutzer für die Scheibe auswählen kann, eine physiologisch zu erwartende Veränderung des Stumpfes. Durch Informationsrückkopplung (Feedback), d.h. durch Auswerten erforderlicher lokaler oder globaler Volumenänderungen am 3D-Schaft-Stumpfmodell desselben Patienten im zeitlichen Verlauf kann die Herstellung eines nachträglich verbesserten Prothesenschafts vereinfacht und verbessert werden. Die Informationsrückkopplung aus sogenannten longitudinalen Verlaufsstudien bzw. Längsschnittstudien über die Veränderung des Patientenstumpfes kann beispielsweise durch optisches Abtasten und/oder anderweitiges Einlesen eines Prothesenschafts durchgeführt werden. Die zum modifizierten 3D-Schaft-Stumpf-Computermodell ermittelten Abweichungen können automatisch von den wissensbasierten Regelsätzen ausgewertet und durch eine erneute Modifizierung des 3D-Schaft-Stumpfmodells angepasst werden. Vorzugsweise kann ein modularer Prothesenschaft vorgesehen sein, der nachträglich durch Hinzufügen, Entfernen oder Verschieben von Prothesenschaft-Teilelementen, z.B. Einlegestücke, Abstandhalter, elastische Matten, Stützelemente oder dergleichen, am oder im Prothesenschaft, auf einfache Art und Weise veränderbar ist, ohne dass ein neuer Schaft erstellt werden muss.

Ferner können an einem Stumpf bildenden Körperteil beispielsweise im zeitlichen Verlauf beim Tragen eines Prothesenschaftes typische Gewebeveränderungen oder Gewebevolumenänderungen auftreten. Es kann daher von Vorteil sein, wenn die selbstlernenden wissensbasierten Regeln unter Berücksichtigung der typischen Gewebeveränderungen oder Gewebevolumenveränderungen ein 3D-Schaft-Stumpfmodell prognostizieren können. Dazu werden die typischen Veränderungen von Stümpfen verschiedener Patienten über die Zeit in longitudinalen Studien ermittelt und ausgewertet. Statistisch signifikante mittlere Volumenänderungen können dann von den wissensbasierten Regelsätzen automatisch bei der Modifizierung eines 3D-Schaft-Stumpf-Modells berücksichtigt werden. Unter Berücksichtigung einer typischen, prognostizierten Gewebeveränderung kann gemäß der Erfindung ein Schaft erstellt werden, welcher vom Patienten länger als gewöhnlich tragbar ist, da er eine Passform aufweist, welche die zukünftige Veränderung des Stumpfes berücksichtigt. Dadurch kann die Tragezeit eines Stumpfes verlängert werden. Auch kann der Behandlungsaufwand für einen Prothesenpatienten reduziert und der Lebensstandard desselben durch seltenere Arztbesuche erhöht werden.

Nach einem weiteren Ausführungsbeispiel kann das Verfahren ein Festlegen einer Referenzebene senkrecht zur Stumpfachse umfassen. Dabei schneidet die Referenzebene einen anatomisch eindeutigen Punkt, bevorzugt eine distale Stelle des Sitzbeins (os ischii) des 3D-Schaft-Stumpfmodells. Die Referenzebene kann zur Ermittelung einer weiteren Ebene - auch Null-Schnitt genannt- in einem vorbestimmten Abstand zu der Referenzebene und parallel zur Referenzebene dienen. Vorzugsweise wird die weitere Ebene in einem Abstand von beispielsweise 5cm zum distalen Ende des Stumpfes hin bestimmt.

Gemäß einem weiteren Ausführungsbeispiel kann die weitere Ebene dazu verwendet werden, den Stumpf in einen proximalen und einen distalen Abschnitt einzuteilen. Dabei kann sowohl die Bestimmung der Referenzebene als auch der weiteren Ebene (Null-Schnitt) und resultierend die Einteilung des 3D-Schaft-Stumpfmodells in proximal-distal vollautomatisch erfolgen.

Gemäß einer weiteren Ausführungsform des erfindungsgemäßen Verfahrens umfasst das Modifizieren eine Volumenkompression, wobei die wissensbasierten Regelsätze wenigstens einen Faktor zur Volumenkompression umfassen. Vorzugsweise wird das 3D-Schaft-Stumpfmodell mittels Kompressionsfaktoren modifiziert, verzerrt und/oder gestaucht. Beispielsweise kann eine Scheibe bzw. eine Schicht, ein Bereich, ein Winkelsektor oder dergleichen modifiziert, verzerrt und/oder gestaucht werden. Ein Volumenkompressionsfaktor ist dabei bevorzugt ein für wenigstens eine der segmentierten Gewebearten spezifischer Kompressionswert, der sich aus den physikalischen Eigenschaften, wie beispielsweise der Kompressibilität der einen segmentierten Gewebeart ergibt. Der Volumenkompressionsfaktor ist bevorzugt ein prozentualer Wert bezogen auf ein anhand des 3D-Schaft-Stumpfmodells ermitteltes Gewebevolumen oder ein entsprechender Absolutwert.

Gemäß einer weiteren Ausführungsform der Erfindung umfasst die Modifizierung eine Volumenexpansion, wobei die wissensbasierten Regelsätze wenigstens einen Faktor zur Volumenexpansion umfassen. In diesem Fall ist der Volumenkompressionsfaktor negativ. Der Faktor zur Volumenänderung ist vorteilhafterweise automatisch oder manuell durch einen Benutzer vorgebbar und/oder veränderbar.

Gemäß einem weiteren Ausführungsbeispiel kann es sich bei dem wenigstens einen Faktor zur Volumenkompression um den Kompressionswert des segmentierten Fettgewebes handeln. Da Fettgewebe eine höhere Kompressibilität als Muskel-, Hautoder Knochengewebe aufweist, entspricht dies den physiologischen Gegebenheiten gut. Im Übrigen kann eine Verringerung des Rechenaufwandes und der Rechenzeit vorteilhaft erreicht werden, wenn lediglich das im Stumpf vorhandene Fettgewebe beim Modifizieren des 3D-Schaft-Stumpfmodells berücksichtigt wird.

Nach einem weiteren Aspekt kann die wenigstens eine Scheibe senkrecht zur Stumpfachse einem proximalen oder einem distalen Bereich des 3D-Schaft-Stumpfmodells zugeordnet werden. Bevorzugt wird im proximalen Bereich des 3D-Schaft-Stumpfmodells ein Modifizieren anhand von als Absolutwert angegebenen Volumenkompressionsfaktoren durchgeführt, welche auf wenigstens einen Winkelsektor angewendet werden, wohingegen im distalen Bereich ein Modifizieren anhand von prozentualen Volumenkompressionsfaktoren durchgeführt wird.

Gemäß einer weiteren Ausführungsform der Erfindung weist die wenigstens eine Scheibe bzw. Schicht eines proximalen Bereiches eine größere Dicke bzw. einen größeren Abstand als die wenigstens eine Scheibe bzw. Schicht eines distalen Bereiches auf. Vorzugsweise kann die Dicke einer Scheibe invers zur Komplexität der in der Scheibe enthaltenen Gewebebereiche und/oder Anatomie ermittelt werden. Beispielsweise wird eine hohe Scheibendicke festgelegt, wenn die Komplexität der enthaltenen Gewebebereiche niedrig ist und umgekehrt. Außerdem kann durch eine angepasste Scheibendicke bzw. Schichtabstand eine vorteilhafte höhere Genauigkeit, beispielsweise im proximalen Bereich des 3D-Schaft-Stumpfmodells erreicht werden. Jede Scheibe oder Schicht enthält Informationen über die Gewebeverteilung in einem im Wesentlichen senkrecht zur Stumpfachse liegenden Volumenelement, jedes Pixel einer zweidimensionalen Darstellung einer Schicht oder Scheibe enthält dreidimensionale Informationen.

Weiter erlaubt das Anwenden von als Absolutwert angegebenen Volumenkompressionsfaktoren im proximalen Bereich größere Scheibendicken bzw. Schichtabstände, da im proximalen Bereich eine Anpassung an gegebene Knochenstrukturen vorteilhaft sein kann. Alternativ werden im distalen Bereich Scheiben größerer Dicke festgelegt, da beispielsweise das distale Ende des Stumpfes bis zur Femur-Spitze keiner Modifikation unterzogen werden braucht. Der Vorteil einer an den Stumpfbereich angepassten Scheibendicke kann in einer Aufwandsreduzierung in Abhängigkeit von der gegebenen Anatomie im 3D-Schaft-Stumpfmodells und der zu erzielenden Modifikation des 3D-Schaft-Stumpfmodells liegen.

Gemäß einer weiteren Ausführungsform wird die wenigstens eine Scheibe in Winkelsektoren eingeteilt, wobei die Winkelsektoren medial kleiner sind als lateral, da medial ein exaktes Modifizieren unter Berücksichtigung der Gewebeverteilung notwendig ist, um beispielsweise das 3D-Schaft-Stumpfmodell an vorhandene Knochenstrukturen besser anpassen zu können oder eine hohe Durchblutung des Stumpfes bei Anliegen des erstellten Schaftes oder allgemein der Nutzung des Schaftes besser gewährleisten zu können. Allgemein kann ein Vorteil von Winkelsektoren vorbestimmter Anzahl mit gleichen und/oder verschiedenen Winkelanteilen in einer schnelleren Anwendung der wissensbasierten Regelsätze oder Volumenkompressionsfaktoren liegen, da im Verfahrensablauf kein weiteres Unterteilen der wenigstens einen Scheibe mehr nötig ist. Durch ein Unterteilen der Scheiben in Winkelsektoren ist ferner eine lokale Modifikation der 3D-Schaft-Stumpfmodells möglich, welche durch Festlegen der Größe eines Winkelsektors vorteilhafterweise individuell an die anatomischen Gegebenheiten eines Stumpfes anpassbar ist.

Nach einer weiteren Ausführungsform der Erfindung erfolgt das Unterteilen der wenigstens einen Scheibe in Winkelsektoren basierend auf einer Auswahl wenigstens einer zuvor bestimmten Winkelsektor-Unterteilung - einem so genannten Template - durch den Benutzer. Templates können in beliebiger Stückzahl durch einen Benutzer manuell vordefiniert oder automatisch erstellt und hinterlegt werden. Die Erstellung der Templates berücksichtigt die Verteilung der verschiedenen Gewebearten in verschiedenen Bereichen des 3D-Schaft-Stumpfmodells. Ferner basiert jedes Template bevorzugt auf wenigstens einer anatomischen Konstitution des 3D-Schaft-Stumpfmodells, wie beispielsweise: adipöser oder muskulöser Patient/Stumpf, männlicher oder weiblicher Patient, linkes Bein oder rechtes Bein, langer Stumpf oder kurzer Stumpf oder der Lage der Scheibe im Stumpf, beispielsweise distaler oder proximaler Stumpfabschnitt. Eine Winkelsektorunterteilung kann auch individuell durch den Benutzer vorgegeben oder variiert werden, um den anatomischen Gegebenheiten eines Stumpfes bestmöglich gerecht zu werden. Bevorzugt wählt der Benutzer eine Winkelsektorunterteilung aus einer vorbestimmten Bibliothek an Templates aus. Zusätzlich kann das erfindungsgemäße Verfahren für den Benutzer eine Vorauswahl wenigstens einer Winkelsektor-Unterteilung treffen, welche auf wenigstens einer anatomischen Konstitution und/oder der Gewebeverteilung des 3D-Schaft-Stumpfmodells basiert. Die anatomische Konstitution kann wenigstens eines der folgenden Kriterien umfassen, die zur Vorauswahl heran gezogen werden können: adipos-muskulös, männlich-weiblich, linkes Bein-rechtes Bein, distal-proximal, langer Stumpf-kurzer Stumpf. Die Vorauswahl kann ferner auf vorherigen Eingaben eines Benutzers basieren oder auf einer Auswertung, wie oft welche WinkelsektorUnterteilung in einem bestimmten Abschnitt eines 3D-Schaft-Stumpfmodells für eine Modifizierung verwendet wurde. Die Vorauswahl kann auch getroffen werden infolge einer Auswertung von longitudinalen Studien über die Volumenveränderungen in verschiedenen Patientenstümpfen.

Nach einer weiteren Ausführungsform der Erfindung wird die Lage der Winkelsektoren der wenigstens einen Scheibe durch Rotation der Winkelsektoren um ihren Scheitelpunkt an die Verteilung wenigstens einer Gewebeart des 3D-Schaft-Stumpfmodells angepasst. Dies erfolgt durch den Benutzer. Außerdem kann es vorteilhaft sein, die Rotation der Winkelsektoren um die Stumpfachse nach Anwenden der wissensbasierten Regelsätze und der Volumenkompressionsfaktoren auf das 3D-Schaft-Stumpfmodell anzuwenden. Die veränderte Reihenfolge der Schrittfolge kann einen zusätzlichen Kontrollschritt für den Benutzer darstellen, indem die ausgeführte Volumenkompression oder Modifikation für einen Bereich des 3D-Schaft-Stumpfmodells mittels einer Parameterlinie im Vergleich zum nicht modifizierten 3D-Schaft-Stumpfmodell anzeigbar ist. Der Orthopädietechniker oder Arzt kann in Abhängigkeit von der ausgeführten Kompression eine Verbesserung der Modifikation durch erneute Rotation der Winkelsektoren und erneutes Anwenden der wissensbasierten Regelsätze oder Volumenkompressionsfaktoren erzielen. Bevorzugt kann die Rotation der Winkelsektoren vollautomatisch erfolgen.

Nach einer weiteren Ausführungsform der Erfindung umfasst das Modifizieren des 3D-Schaft-Stumpfmodells anhand von wissensbasierten Regelsätzen ein Anpassen auf der Grundlage der äußeren Form, insbesondere der Krümmungen, des 3D-Schaft-Stumpfmodells. Ferner umfasst das Modifizieren vorzugsweise ein Anpassen an wesentliche Knochenstrukturen, vorzugsweise der Ramus ossis ischii und der Tuber ischiadicum, da dieser Bereich am Becken für einen Überknieamputierten (Transfemuralamputierten) schafttragenden Patienten bei Fehlanpassung des Prothesenschaftes andernfalls zu Schmerzen führen kann. Bevorzug erfolgt die Anpassung an Knochenstrukturen anhand von als Absolutwert angegebenen Volumenkompressionswerten. Vorteilhafterweise liefert das 3D-Schaft-Stumpfmodell dazu Informationen über die Verteilung, Lage und Beschaffenheit dieser Knochen und des darüberliegenden Gewebes, was das Anpassen vereinfacht. Bevorzugt erfolgt die Anpassung des 3D-Schaft-Stumpfmodells an wesentliche Knochenstrukturen vollautomatisch oder durch eine Interaktion mit einem Benutzer.

Nach einer weiteren Ausführungsform wird beim Modifizieren des 3D-Schaft-Stumpfmodells eine Volumenänderung des Schaft-Stumpf-Modells im Wesentlichen parallel zur Stumpf-Achse - eine Längskompression - anhand der wissensbasierten Regelsätze berücksichtigt. Vorteilhafterweise kann für eine Berücksichtigung der Längskompression das Körpergewicht des Patienten eingehen, um möglichst eine punktuelle oder regionale Erhöhung der Druckbelastung des Stumpfes, besonders im distalen Bereich des Stumpfes bei Anliegen des zu erstellenden Schaftes zu vermeiden.

Nach einer weiteren vorteilhaften Ausführungsform des erfindungsgemäßen Verfahrens wird beim Modifizieren des 3D-Schaft-Stumpfmodells eine Oberflächenglättung des modifizierten 3D-Schaft-Stumpfmodells durchgeführt, wobei die Oberflächenglättung zwischen zwei benachbarten Scheiben erfolgt. Während gemäß den manuellen Herstellungsverfahren des Standes der Technik für einen Prothesenschaft eine glatte, stufenlose Schaft-Oberfläche erzielt werden sollte, was einer globalen Glättung der Schaft-Oberfläche entspricht, kann es sich vorteilhaft auf die Trageeigenschaften des zu erstellenden Schaftes auswirken, das modifizierte 3D-Schaft-Stumpfmodell alternativ einer lokalen, also regional begrenzten Oberflächenglättung zu unterziehen. Dabei werden bevorzugt die Außenflächen von benachbarten, modifizierten Bereichen des 3D-Schaft-Stumpfmodells, beispielsweise zwei benachbarten in Winkelsektoren unterteilten Scheiben im Übergangsbereich aneinander angepasst, nachdem wenigstens ein Winkelsektor wenigstens einer der beiden Scheiben anhand wissensbasierter Regelsätze in Form von Volumenkompressionsfaktoren modifiziert wurde. Dazu kann beispielsweise ein räumliches Interpolationsverfahren oder dergleichen dienen. Dadurch ergeben sich lokale Unebenheiten in der 3D-Schaft-Stumpfmodell-Oberfläche. Diese können sich vorteilhaft auf den Tragekomfort, den hydrostatischen Ansatz und dadurch auf die Hafteigenschaften des zu erstellenden Stumpfes auswirken. Es kann ferner vorteilhaft sein, sowohl eine lokale als auch eine globale Oberflächenglättung in verschiedenen Bereichen des 3D-Schaft-Stumpfmodells durchzuführen. Die Oberflächenglättung erfolgt bevorzugt vollautomatisch.

Das Modifizieren hat vorzugsweise zur Folge, dass der Stumpf während des Tragens eines nach dem 3D-Schaft-Stumpfmodell hergestellten Schaftes und bei Bewegung des Stumpfes unter unterschiedlichen Bedingungen ein Volumen aufweist, das sich von dem Stumpfvolumen im entspannten Zustand und ohne einen Schaft zu tragen, unterscheidet.

Ist das Modifizieren des 3D-Stumpfmodells abgeschlossen, erfolgt das Herstellen, insbesondere Fräsen, um das 3D-Schaft-Stumpfmodell schnell und kostengünstig als Schaft einsetzen zu können.

Nach einer weiteren Ausführungsform der Erfindung wird das 3D-Schaft-Stumpfmodell durch Fräsen und/oder Schleifen und/oder Drehen und/oder Laserschneiden und/oder Tiefziehen hergestellt. Vorteilhafterweise wird die für ein jeweils verwendetes Schaftmaterial geeignete und schnellste Bearbeitungsmethode oder eine Kombination bekannter geeigneter Bearbeitungsmethoden gewählt.

Nach einer weiteren Ausführungsform der Erfindung kann ein anatomischer Fehler beim 3D-Schaft-Stumpfmodell unter Einsatz wenigstens einer vorgegebenen 3D-Schablone korrigiert werden. 3D-Schablonen sind vordefinierte virtuelle Modell-Schäfte oder Modell-Teilschäfte, welche spezifische Informationen über eine Stumpf-Anatomie inherent berücksichtigen. Vorteilhafterweise können solche 3D-Schablonen durch Probeschäfte bei Versuchspatienten ermittelt werden und dann automatisch anatomische Fehler bei 3D-Schaft-Stumpfmodellen korrigieren. Die Schablonen werden bevorzugt in einer Datenbank abgelegt und werden entsprechend den anatomischen Gegebenheiten eines Patienten ausgewählt und im Rahmen der Modifikation des 3D-Schaft-Stumpf-Modells verwendet.

Nach einer weiteren Ausführungsform der Erfindung kann ein zwischen einem Prothesenfuß und dem Schaft liegendes Knie-Waden-Passteil basierend auf vom unversehrten zweiten Bein ermittelten 3D-Bilddaten dimensioniert werden. Vorzugsweise kann eine CT-Aufnahme eine Vergleichsinformation, beispielsweise eine gespiegelte Symmetrieinformation, vom unversehrten zweiten Bein auch für die Konstruktion und das Anpassen der Prothese und/oder des Prothesenschaftes verwendet werden.

Einer weiterer Aspekt der Erfindung betrifft ein System zur Erstellung eines 3D-Schaft-Stumpfmodells zur Herstellung eines Prothesenschaftes zur Verbindung eines einen Stumpf bildenden Körperteils mit einer Prothese: eine Akquiriereinheit zum Akquirieren von dreidimensionalen Bilddaten des den Stumpf bildenden Körperteils, das mehrere Gewebearten umfasst; eine Segmentiereinheit zum Segmentieren der 3D-Bilddaten von der Akquiriereinheit zur Bestimmung der Verteilung von wenigstens einer Gewebeart des Stumpfs; eine Rekonstruiereinheit zum Rekonstruieren eines 3D-Schaft-Stumpfmodells anhand der segmentierten 3D-Bilddaten von der Segmentiereinheit, das Geometrie des Stumpfes und Verteilung der wenigstens einen segmentierten Gewebeart des Stumpfes beschreibt; eine Festlegeeinheit zum Festlegen anhand des 3D-Schaft-Stumpfmodells wenigstens einer Stumpfachse; eine Unterteileinheit zum Unterteilen wenigstens eines Bereiches des 3D-Schaft-Stumpfmodells in wenigstens eine Scheibe bestimmter Dicke senkrecht zur Stumpfachse und zum Unterteilen der wenigstens einen Scheibe in Winkelsektoren und eine Modifiziereinheit zum Modifizieren des 3D-Schaft-Stumpfmodells von der Rekonstruiereinheit anhand von wissensbasierten Regelsätzen zur optimalen Anpassung des 3D-Schaft-Stumpfmodells an den Stumpf, wobei die wissensbasierten Regelsätze die in dem 3D-Schaft-Stumpfmodell enthaltenen Informationen über Geometrie des Stumpfes und/oder Verteilung der wenigstens einen segmentierten Gewebeart berücksichtigen und eine oder mehrere Regeln umfassen, die eine oder mehrere Eigenschaften der wenigstens einen segmentierten Gewebeart verwenden.

Ein weiterer Aspekt der Erfindung betrifft eine Vorrichtung zur Interaktion eines Benutzers mit einem 3D-Schaft-Stumpf-Computermodell zur Modifizierung des 3D-Schaft-Stumpf-Computermodells, das die Oberflächenform und räumliche Gewebeverteilung eines Stumpfes beschreibt. Die Vorrichtung ist ausgelegt, das 3D-Schaft-Stumpf-Computermodell in Abschnitte zu unterteilen und umfasst folgende Elemente: ein Display, welches ausgelegt ist, die Oberflächenform und Gewebeverteilung in einem Abschnitt des 3D-Schaft-Stumpf-Computermodells anzuzeigen, ein erstes Auswahlmodul, welches dem Benutzer ermöglicht, einen Abschnitt des 3D-Schaft-Stumpf-Computermdells zur Anzeige an dem Display auszuwählen und ein zweites Auswahlmodul, das dem Benutzer ermöglicht, wenigstens eine vorab festgelegte räumliche Verteilung einer Modifizierung der Oberflächenform in dem Abschnitt auszuwählen. Die Vorrichtung ist dabei ausgelegt, entsprechend der ausgewählten räumlichen Verteilung die Oberflächenform in dem Abschnitt zu modifizieren.

Die Interaktion eines Benutzers mit einem 3D-Schaft-Stumpf-Computermodell umfasst dabei bevorzugt jede Wechselwirkung oder Eingabe eines Benutzers anhand einem der Auswahlmodule, auf der die Modifizierung des 3D-Schaft-Stumpf Computermodells basiert, wie beispielsweise das Betrachten des 3D-Schaft-Stumpf-Computermodells oder bestimmter Ausschnitte durch den Benutzer an einem Display, das Auswählen bestimmter Modell-Abschnitte zur Anzeige an dem Display, das Markieren von anatomischen Punkten in einem Abschnitt, das Auswählen einer vorab festgelegten Verteilung einer Modifizierung der Oberflächenform, usw..

Das Display kann vorzugsweise das Display eines Computerbildschirms sein, welches geeignet ist, die dreidimensionalen Informationen über die räumliche Verteilung der Gewebearten und die Oberflächenform des abgebildeten Stumpfes darzustellen.

Das erste Auswahlmodul umfasst bevorzugt ein Interaktionsmodul wie beispielsweise eine Computermaus, eine Tastatur oder ein Touch-Screen, mittels welcher der Benutzer am Display angezeigte Abschnitte des 3D-Schaft-Stumpf-Computermodells auswählen und einzeln und vergrößert zur Anzeige bringen kann. Die Darstellung des ausgewählten Abschnittes erfolgt derart, dass dem Benutzer die Oberflächenform und die räumliche Verteilung der in dem Abschnitt enthaltenen Gewebearten angezeigt werden. Vorteilhafterweise handelt es sich bei der Darstellung um eine Aufsicht auf den Abschnitt, aber auch perspektivische Ansichten sind möglich. Dem Benutzer kann zusätzlich auch die Lage des ausgewählten Abschnittes innerhalb des 3D-Schaft-Stumpfmodells angezeigt werden, sodass er die Lage bei der Auswahl einer räumlichen Verteilung einer Modifizierung berücksichtigen kann.

Das zweite Auswahlmodul umfasst ebenfalls eine Eingabevorrichtung, die es dem Benutzer ermöglicht, eine vorab festgelegte räumliche Verteilung einer Modifizierung der Oberflächenform für den ausgewählten Abschnitt auszuwählen. Bevorzugt wählt der Benutzer dazu aus einer Reihe von über das Display angezeigten räumlichen Verteilungen aus. Die räumlichen Verteilungen der Modifizierungen sind derart ausgelegt, dass sie die räumliche Gewebeverteilung eines Abschnittes berücksichtigen. Ein Satz vorbestimmter räumlicher Verteilungen kann beispielweise in einer Datenbank in einem Datenbankmodul hinterlegt sein, auf welche das zweite Auswahlmodul zugreifen kann. Die Einträge der Datenbank werden bevorzugt manuell durch einen Benutzer generiert, überwacht und geändert. Vorteilhafterweise kann die Vorrichtung auch den Satz an räumlichen Verteilungen der Modifizierung automatisch anpassen.

Entsprechend der ausgewählten räumlichen Verteilung der Modifizierung modifiziert die Vorrichtung die Oberflächenform des ausgewählten Abschnitts. Bevorzugt handelt es sich bei der Modifizierung um eine Volumenänderung in dem Abschnitt. Die Modifizierung kann auch in einer reinen Formänderung, oder einer Verzerrung oder Stauchung des Abschnittes liegen. Die Modifizierung des 3D-Schaft-Stumpfmodells erfolgt vorteilhafterweise derart, dass eine ideale Passform des zu erstellenden Schaftes an den vorliegenden Stumpf erreicht wird.

Die Ausführungsform ermöglicht einem Benutzer einen vereinfachten Bearbeitungsablauf bei der Modifizierung eines 3D-Schaft-Stumpf-Computermodells, da der Benutzer in der Lage ist, Abschnitte des 3D-Schaft-Stumpfmodells individuell zur Anzeige bringen und entsprechend der Gewebeverteilung durch Auswählen einer Modifizierverteilung modifizieren kann. Ein Vorteil liegt darin, dass der Benutzer vorbestimmte räumliche Verteilungen für die Modifizierung eines jeden Abschnittes angeboten werden, aus denen er eine passende auswählen kann. Dies verringert den Aufwand und die benötigten Eingaben des Benutzers beim Modifizieren eines 3D-Schaft-Stumpfmodells, die Kosten und die Dauer des Anpassungsprozesses. Zusätzlich erhöht sich die Qualität der damit erstellten Schäfte.

Gemäß einer weiteren Ausführungsform der Erfindung ist die Vorrichtung ausgelegt dem Benutzer zu ermöglichen, eine Stumpfachse anhand des 3D-Schaft-Stumpf-Computermodells festzulegen und das 3D-Schaft-Stumpf-Computermodell in Abschnitte zu unterteilen, welche an der Stumpfachse ausgerichtet sind.

Zur Festlegung einer Stumpfachse in einem 3D-Schaft-Stumpfmodel wählt der Benutzer zwei Abschnitte, beispielsweise Schnittbilder des Stumpfes, welche mittels eines medizinischen Bildgebungsverfahrens wie CT oder MRT aufgenommen wurden, aus und wählt mit Hilfe eines der Auswahlmodule einen definierten anatomischen Punkt in den Abschnitten aus. Dadurch legt der Benutzer zwei Punkte fest, durch welche die Stumpfachse verläuft. Durch die Interaktion des Benutzers bei der Festlegung der Stumpfachse kann die Vorrichtung, besondere Gegebenheiten eines Stumpfes individuell berücksichtigen. Unter Berücksichtigung der Lage der Stumpfachse berechnet die Vorrichtung automatisch Schnittbilder, die im Wesentlichen senkrecht zu der Stumpfachse verlaufen. Anhand dieser Abschnitte erfolgt die Modifizierung des 3D-Schaft-Stumpfmodells.

Nach einer weiteren Ausführungsform der Erfindung handelt es sich bei den Abschnitten anhand derer die Modifizierung stattfindet um im Wesentlichen senkrecht zur Stumpfachse liegende Scheiben. Die Scheiben können eine beliebige Dicke aufweisen. Beispielsweise können alle Scheiben dieselbe Dicke aufweisen. Bevorzugt weisen die Scheiben unterschiedliche Dicken, beispielsweise im proximalen Bereich eine größere Dicke als im distalen Bereich des 3D-Schaft-Stumpf-Computermodells auf, da ein Modifizieren im proximalen Bereich auf Grundlage der dort vorhandenen Knochenstrukturen erfolgt, was eine gröbere Einteilung zulässt. Die Vorrichtung ist vorteilhafterweise ausgelegt, dem Benutzer zu ermöglichen, die Dicke einer Scheibe individuell zu bestimmen. Zeigt ein 3D-Schaft-Stumpfmodell Besonderheiten in der Gewebestruktur auf, so können diese schon bei der Einteilung des 3D-Schaft-Stumpf-Computermodells individuell berücksichtigt werden. Die Dicke einer Scheibe kann beispielsweise durch Eingabe eines Wertes über eine Tastatur durch den Benutzer erfolgen oder durch eine Auswahl eines Wertes aus einer Reihe zur Auswahl gestellter Werte bestimmt werden.

Nach einer weiteren Ausführungsform der Erfindung umfasst die vorab festgelegte räumliche Verteilung eine Unterteilung des Abschnittes in wenigstens einen Unterbereich, wobei jedem Unterbereich wenigstens ein Wert zugeordnet ist, auf dem die Stärke der Modifizierung der Oberflächenform basiert. Bevorzugt handelt es sich bei den Unterbereichen um derart geformte Unterbereiche, die die Geometrie des 3D-Schaft-Stumpf-Computermodells berücksichtigen. Bevorzugt ist für jeden der Unterbereiche wenigstens ein fester Wert festgelegt, anhand welchem die Stärke der Modifizierung in diesem Unterbereich bestimmt werden kann. Die Einteilung eines Abschnittes in Unterbereiche erzielt bei der Modifizierung des 3D-Schaft-Stumpf-Computermodells einen verringerten Rechenaufwand, da für jeden Unterbereich lediglich eine feste Anzahl von Werten vorliegt, welche die Modifizierung in diesem Unterbereich charakterisiert. Durch eine feine Unterteilung der Abschnitte in viele kleine Unterbereiche mit entsprechenden Werten kann dennoch die Genauigkeit der Modifizierung erhöht werden, beispielsweise dann, wenn die anatomischen Gegebenheiten des 3D-Schaft-Stumpf-Computermodells dieses verlangen.

Nach einer weiteren Ausführungsform der Erfindung ist die Vorrichtung ausgelegt, auf Basis des Wertes und der Gewebeverteilung in dem Unterbereich eine Volumenänderung einer der in dem Unterbereich enthaltenen Gewebearten abzuleiten und anhand der Volumenänderung die Stärke der Modifizierung der Oberflächenform zu bestimmen.

Nach einer weiteren Ausführungsform der Erfindung umfassen die Gewebearten in dem 3D-Schaft-Stumpf-Computermodell Fett, Muskel, Haut und/oder Knochen. Der Wert der Volumenänderung wenigstens einer der enthaltenen Gewebearten kann dabei die prozentuale Volumenänderung der jeweiligen Gewebeart oder eine absolute Volumenänderung angeben. Der Wert einer Volumenänderung wenigstens einer der enthaltenen Gewebearten kann bevorzugt auf der Kompressibilität der jeweiligen Gewebeart basieren.

Nach einer weiteren Ausführungsform der Erfindung ermöglicht das zweite Auswahlmodul dem Benutzer, den Wert für die Modifizierung in wenigstens einem Unterbereich manuell zu verändern. Dies kann bevorzugt durch eine Abfrage durch die Vorrichtung erfolgen oder durch Eingabe eines veränderten Wertes durch den Benutzer. Von Vorteil ist, dass der Benutzer nicht an die ihm zur Auswahl stehenden Vorschläge für die Modifikation gebunden ist, aber eine Anpassung der Vorschläge für eine Modifikation lokal für einen Winkelbereich durchführen kann, wenn die anatomischen Gegebenheiten des 3D-Schaft-Stumpf-Computermodells dies verlangen. Große Teile einer vorab festgelegten räumlichen Verteilung der Modifizierung können so beibehalten werden. Bevorzugt kann so ein weiterer Kontrollschritt durch den Benutzer erfolgen.

Nach einer weiteren Ausführungsform der Erfindung umfasst die räumliche Verteilung der Modifizierung Unterbereiche, die Winkelsektoren bilden, welche radial ausgehend von der Stumpfachse angeordnet sind. Dies entspricht einer optimalen Berücksichtigung der angenäherten Zylindersymmetrie eines Stumpfes bei der Modifizierung des 3D-Schaft-Stumpf-Computermodells. Bei besonders kurzen Stümpfen kann es vorteilhaft sein, auf eine andere Unterteilung oder gar eine andere Form der Abschnitte auszuweichen, die beispielsweise einer Kugelsymmetrie entlehnt sind.

Nach einer weiteren Ausführungsform der Erfindung ermöglicht die Vorrichtung dem Benutzer, über das zweite Auswahlmodul, die räumliche Ausrichtung des Winkelsektors durch Drehen um die Stumpfachse manuell zu verändern. Bevorzugt wird dazu die ausgewählte räumliche Verteilung mit dem ausgewählten Abschnitt überlagert und die Überlagerung dem Benutzer angezeigt. Dieser kann nun die Verteilung über dem Abschnitt drehen, bis eine optimale Zuordnung der verschiedenen Gewebearten zu den Winkelsektoren erfolgt ist. Bevorzugt kann der Benutzer eine weitere Drehung der Verteilung über dem Abschnitt nach einer durchgeführten Modifizierung der Oberflächenform des Abschnittes durchführen, wenn er mit dem Ergebnis der Modifizierung noch nicht abschließend zufrieden ist.

Die Modifizierung der Oberflächenform ist nach einer weiteren Ausführungsform der Erfindung derart ausgestaltet, dass die Form der die Außenfläche des 3D-Schaft-Stumpf-Computermodells bildenden Fläche des Unterbereiches im Wesentlichen erhalten bleibt. Bevorzugt erfolgt dazu die Modifizierung eines Unterbereiches derart, dass die die Außenfläche des 3D-Schaft-Stumpf-Computermodells bildende Seite eines Winkelsektors gemäß der Stärke der Volumenänderung radial verschoben wird und die Übergangsbereiche zwischen den die Außenflächen bildenden Seiten zweier benachbarter Winkelsektoren einer Glättung unterzogen werden. Die Stärke der Volumenänderung des Winkelsektors ergibt sich aus dem Volumenänderungsfaktor für wenigstens eine in dem Winkelsektor enthaltene Gewebeart.

Gemäß einer weiteren Ausführungsform der Erfindung ist das zweite Auswahlmodul derart ausgelegt, dass die vorab festgelegten räumlichen Verteilungen, aus denen der Benutzer für den jeweiligen Abschnitt auswählen kann, abhängen von der Lage des Abschnittes innerhalb des Stumpfes, der Gewebeverteilung des Abschnittes und/oder physiologischen oder anatomischen Eigenschaften des Stumpfes bzw. des Patienten. Dies umfasst bevorzugt eine Einteilung der verschiedenen räumlichen Verteilungen der Modifizierung in folgende Kategorien: distal oder proximal, langer Stumpf oder kurzer Stumpf, linkes Bein oder rechtes Bein, muskulöser oder adipöser Stumpf, männlich oder weiblich, usw. Die räumlichen Verteilungen werden bevorzugt in den genannten Kategorien in der Datenbank abgelegt. Durch Auswertung der Auswahl eines Abschnittes durch den Benutzer und/oder der darin enthaltenen Gewebeverteilung oder anderer Kenngrößen des 3D-Schaft-Stumpf-Computermodells, wie beispielsweise der Länge des Stumpfes kann die Vorrichtung eine Vorauswahl der räumlichen Verteilungen für den Benutzer treffen und diesem lediglich die Vorauswahl präsentieren, aus welcher der Benutzer dann auswählt. Die intelligente Vorauswahl und/oder Filterung der räumlichen Verteilungen durch die Vorrichtung kann den Anpassungsprozess weiter beschleunigen.

Nach einer weiteren Ausführungsform der Erfindung umfasst die Vorrichtung auch ein Datenbankmodul, in dem die vorab festgelegten räumlichen Verteilungen abgelegt sind und die Vorrichtung ausgelegt ist, basierend auf einer Analyse von vorausgegangenen Modifizierungen des 3D-Schaft-Stumpf-Computermodells anderer und/oder desselben Patienten den Satz der räumlichen Verteilungen in der Datenbank anzupassen. Die Analyse umfasst bevorzugt, wie häufig eine räumliche Verteilung für einen Abschnitt durch einen Benutzer ausgewählt wurde. Damit erstellt die Vorrichtung Listen der räumlichen Verteilungen, welche die Wichtigkeit einer räumlichen Verteilung angeben. Unwichtige, also kaum oder gar nicht benutzte räumliche Verteilungen werden nach Ablauf einer festgesetzten Frist aus der Datenbank gelöscht, wohingegen wichtige, also häufig benutzte räumliche Verteilungen dem Benutzer bevorzugt zur Auswahl gestellt werden. Durch diesen Selbstlernprozess kann die Modifizierung eines 3D-Schaft-Stumpf-Computermodells ständig optimiert werden. Weiter kann die Analyse umfassen, wie häufig und in welchem Ausmaß eine räumliche Verteilung nach der Auswahl für einen Abschnitt durch den Benutzer verändert wurde. Die Veränderung beispielsweise der Kompressionswerte kann registriert und ausgewertet werden. Entsprechend dieser Anpassungen durch den Benutzer können entweder schon bestehende räumliche Verteilungen angepasst oder neue räumliche Verteilungen in der Datenbank erstellt werden.

Gemäß einer weiteren Ausführungsform der Erfindung ist das zweite Auswahlmodul derart ausgelegt, dass die vorab festgelegten räumlichen Verteilungen, aus denen der Benutzer für den jeweiligen Abschnitt auswählen kann, eine physiologisch zu erwartende Veränderung des Stumpfes berücksichtigen. Die physiologisch zu erwartenden Veränderungen des Stumpfes umfassen dabei die Reduktion des Muskelgewebes, welches nach einer Amputation einer Inaktivitätsatrophie unterliegt, eine Erhöhung des Fettanteils im Stumpf, usw. Diese Veränderungen können typischerweise bei Amputationspatienten häufig auftreten. Durch Vermessen vieler Patientenstümpfe im Rahmen von longitudinalen Studien können signifikante mittlere Stumpfveränderungen ermittelt werden. Die longitudinalen Studien können einen Patientenstumpf beispielsweise unmittelbar nach der Amputation und dann in einem bestimmten zeitlichen Intervall vermessen. Die daraus gewonnenen Informationen über eine im Mittel zu erwartende Veränderung des Stumpfes werden über einen Informationsrückfluss (Feedback) an die Vorrichtung übertragen und von der Vorrichtung ausgewertet. Beispielsweise kann nach Auswahl einer räumlichen Verteilung für einen Abschnitt durch einen Benutzer eine Abfrage erfolgen, ob für diesen Abschnitt die zu erwartende Veränderung berücksichtigt werden soll. Bei Bestätigung durch den Benutzer werden die Kompressionswerte in den Winkelsektoren angepasst. Alternativ können räumliche Verteilungen in der Datenbank abgelegt sein, die die Information über die zu erwartende Veränderung schon berücksichtigen, beispielsweise durch eine automatische Erhöhung der Kompressionswerte in den einzelnen Winkelsektoren, sodass eine Abfrage überflüssig wird. Außerdem kann das 3D-Schaft-Stumpf-Computermodell nach einer abschnittsweisen Modifizierung unter Berücksichtigung der Informationen über die zu erwartende Veränderung global angepasst werden. Vorteile einer Berücksichtigung der zu erwartenden Veränderung des Stumpfes können in einer Verlängerung der Tragezeit des derart hergestellten Schaftes liegen, da nicht die aktuelle Form des Stumpfes, sondern eine zu erwartende Form des Stumpfes für die Herstellung des Schaftes heran gezogen wird. Durch leichte Abweichungen von einer aktuell optimalen Passform hin zu einer zukünftig optimalen Passform kann die Tragezeit eines Schaftes erheblich erhöht und auch der Aktivitätsgrad sowie der Lebensstandard eines Patienten erhöht werden.

Ein weiterer Aspekt der Erfindung betrifft ein Verfahren zur Interaktion eines Benutzers mit einem 3D-Schaft-Stumpf-Computermodell zur Modifizierung des 3D-Schaft-Stumpf-Computermodells, das die Oberflächenform und räumliche Gewebeverteilung eines Stumpfes beschreibt. Das Verfahren umfasst die folgenden Schritte: Unterteilen des 3D-Schaft-Stumpf-Computermodells in Abschnitte, Auswählen eines Abschnitts des 3D-Schaft-Stumpf-Computermodells zur Anzeige an einem Display durch den Benutzer, Anzeigen der Oberflächenform und Gewebeverteilung des ausgewählten Abschnittes an einem Display, Auswählen wenigstens einer vorab festgelegten räumlichen Verteilung einer Modifizierung der Oberflächenform in dem Abschnitt durch einen Benutzer, und Modifizieren der Oberflächenform in dem Abschnitt entsprechend der ausgewählten räumlichen Verteilung.

Gemäß einer weiteren Ausführungsform des erfindungsgemäßen Verfahrens wird anhand des 3D-Schaft-Stumpf-Computermodells eine Stumpfachse festgelegt und das 3D-Schaft-Stumpf-Computermodell in Abschnitte unterteilt, welche an der Stumpfachse ausgerichtet sind.

Gemäß einer weiteren Ausführungsform des erfindungsgemäßen Verfahrens umfasst die vorab festgelegte räumliche Verteilung eine Unterteilung des Abschnittes in wenigstens einen Winkelsektor, der radial ausgehend von der Stumpfachse angeordnet ist, wobei jedem Winkelsektor wenigstens ein Wert zugeordnet ist, auf dem die Stärke der Modifizierung basiert.

Gemäß einer weiteren Ausführungsform der Erfindung wird die räumliche Ausrichtung des Winkelsektors durch Drehen um die Stumpfachse durch einen Benutzer manuell verändert.

Gemäß einer weiteren Ausführungsform der Erfindung kann der Wert manuell durch den Benutzer geändert werden.

Gemäß einer weiteren Ausführungsform der Erfindung wird beim Modifizieren die Form der die Außenfläche des 3D-Schaft-Stumpf-Computermodells bildenden Fläche des Unterbereiches im Wesentlichen erhalten.

Ein weiterer Aspekt der Erfindung betrifft ein Computerprogramm, das angepasst ist, um das erfindungsgemäße Verfahren durchzuführen. Das Computerprogramm ist insbesondere geeignet, ein Verfahren zur Interaktion eines Benutzers mit einem 3D-Schaft-Stumpf-Computermodell zur Modifizierung des 3D-Schaft-Stumpf-Computermodells auszuführen. Das Computerprogramm ist bevorzugt auf einem computerlesbaren Medium gespeichert. Das Computerprogramm kann als Computerprogrammprodukt in Form einer CD, DVD oder eines anderen beliebigen transportablen Datenspeichers, beispielsweise eines USB-Sticks vorliegen. Das Computerprogramm kann alternativ als Computerprogrammprodukt auf der Festplatte eines Rechners abgelegt sein. Alternativ kann das Computerprogramm zentral auf einem Server abgelegt und via Internet und/oder via lokalem Netzwerk durch einen Benutzer aufrufbar und/oder ausführbar sein. Das Computerprogrammprodukt ist vorteilhafterweise geeignet, das erfindungsgemäße Verfahren auszuführen, wenn das Computerprogramm auf einem Computer ausgeführt wird. Der Programmcode umfasst bevorzugt ausführbaren Code und/oder Quellcode des erfindungsgemäßen Computerprogramms.

Ein weiterer Aspekt der Erfindung betrifft ein System zur Interaktion eines Benutzers mit einem 3D-Schaft-Stumpf-Computermodell zur Modifizierung des 3D-Schaft-Stumpf-Computermodells. Das System umfasst: eine Einleseeinheit zum Einlesen von 3D-Bilddaten des Stumpfes, eine Segmentiereinheit zum Segmentieren der 3D-Bilddaten zur Bestimmung der räumlichen Gewebeverteilung des Stumpfs, eine Rekonstruiereinheit zum Rekonstruieren eines 3D-Schaft-Stumpf-Computermodells anhand der segmentierten 3D-Bilddaten, welches Oberflächenform und räumliche Gewebeverteilung des Stumpfes beschreibt, eine Vorrichtung zur Interaktion mit einem Benutzer, welche das 3D-Schaft-Stumpf-Computermodell unter Berücksichtigung von Eingaben des Benutzers modifiziert, und eine Ausgabeeinheit, welche das modifizierte 3D-Schaft-Stumpf-Computermodell zur Weiterverwendung für die Herstellung eines Prothesenschaftes ausgibt.

### KURZBESCHREIBUNG DER ZEICHNUNGEN

Nachstehend werden weitere bevorzugte beispielhafte Ausführungsformen der Erfindung anhand schematischer Zeichnungen näher erläutert. Dabei zeigen:
- Fig. 1: ein Flussdiagramm nach einem Ausführungsbeispiel der Erfindung;
- Fig. 2: Schichtaufnahme in Frontalebenen nach einem Ausführungsbeispiel der Erfindung;
- Fig. 3a: eine Scheibe segmentierter Gewebearten nach einem Ausführungsbeispiel der Erfindung;
- Fig. 3b: eine Scheibe segmentierter Gewebearten nach einem Ausführungsbeispiel der Erfindung;
- Fig. 4: eine Erstellung eines Schaftes nach einem Ausführungsbeispiel der Erfindung;
- Fig. 5: Lagen von Achsen nach einem Ausführungsbeispiel der Erfindung;
- Fig. 6: mehrere Scheiben nach einem Ausführungsbeispiel der Erfindung;
- Fig. 7: eine Scheibe mit Winkelsektoren nach einem Ausführungsbeispiel der Erfindung;
- Fig. 8a: ein Ablaufdiagramm nach einem Ausführungsbeispiel der Erfindung;
- Fig. 8b: ein Ablaufdiagramm nach einem Ausführungsbeispiel der Erfindung;
- Fig. 8c: ein Ablaufdiagramm nach einem Ausführungsbeispiel der Erfindung;
- Fi. 9: eine Darstellung einer Oberflächenglättung des komprimierten 3D-Schaft-Stumpfmodells nach einem Ausführungsbeispiel der Erfindung;
- Fig. 10: eine schematische Kraftverteilung auf den Stumpf bei Anliegen des zu erstellenden Schaftes nach einem Ausführungsbeispiel der Erfindung;
- Fig. 11: ein Interaktionsschema eines Benutzers mit einem 3D-Schaft-Stumpf-Computermodell nach einem Ausführungsbeispiel der Erfindung;
- Fig. 12: ein Interaktionsschema eines Benutzers mit einem 3D-Schaft-Stumpf-Computermodell nach einem Ausführungsbeispiel der Erfindung;
- Fig. 13: eine schematische Darstellung einer Datenbank mit Templates nach einem Ausführungsbeispiel der Erfindung.

### BESCHREIBUNG BEVORZUGTER AUSFÜHRUNGSFORMEN

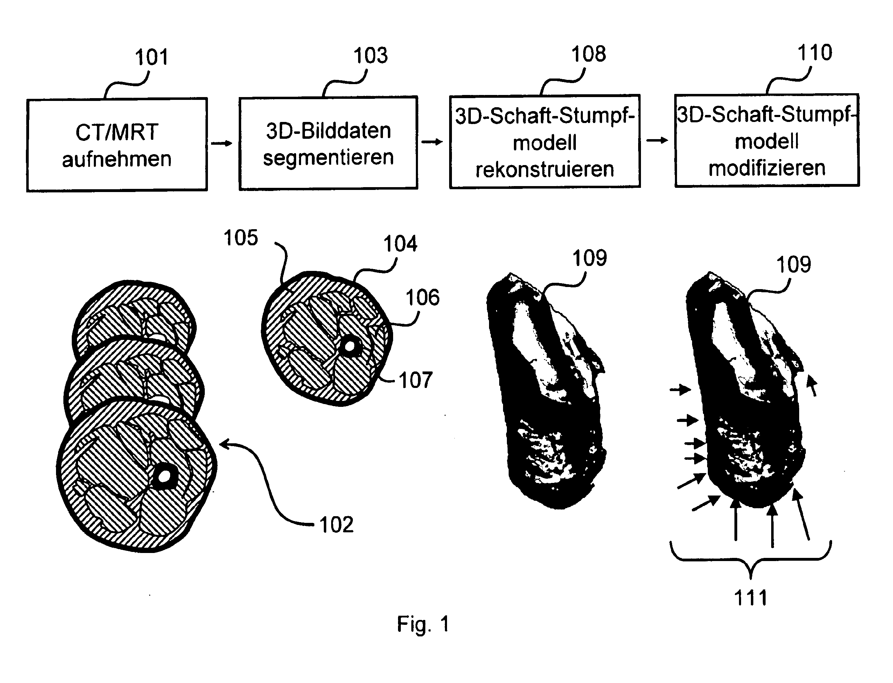

Fig. 1 zeigt ein Flussdiagramm nach einem Ausführungsbeispiel der Erfindung, das folgende Schritte (in dieser Reihenfolge) umfasst: Schritt 101 (Akquiriereinheit) CT-und/oder MRT-Schichtaufnahmen 102 zum Akquirieren von 3D-Bilddaten des Stumpfes, die in einer Wolke aus Dichtepunkten resultieren; Schritt 103 (Segmentiereinheit) 3D-Bilddaten segmentieren zur Bestimmung einer Verteilung von Gewebearten des Stumpfes, die Haut 104, Fett 105, Muskeln 106 und Knochen 107 umfassen; Schritt 108 (Rekonstruktionseinheit) Rekonstruieren eines 3D-Schaft-Stumpfmodells 109 anhand der segmentierten 3D-Bilddaten und Schritt 110 (Modifiziereinheit) Modifizieren des 3D-Schaft-Stumpfmodells basierend auf der Kompressibilität der segmentierten Gewebearten 105 bis 107. Die Pfeile 111 symbolisieren manuell oder automatisch ablaufende Modifizierungen, Verzerrungen und/oder Stauchungen des 3D-Stumpfmodells, wobei die Länge der Pfeile qualitativ für das Maß der Modifizierung steht.

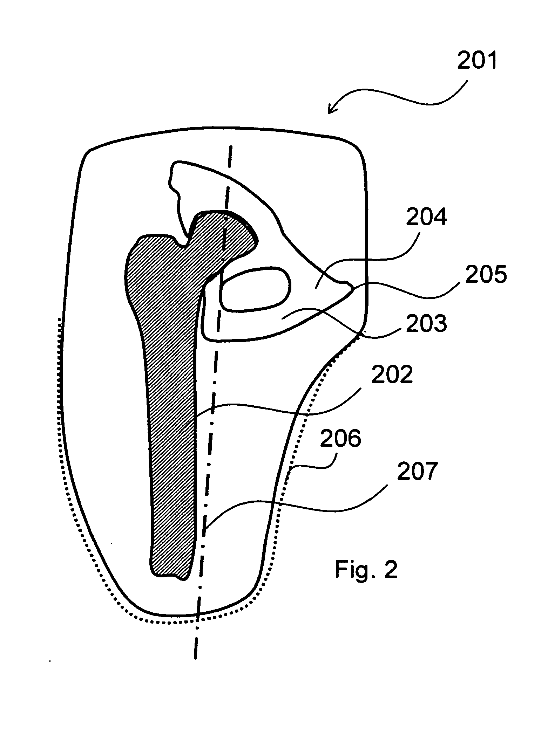

Fig. 2 zeigt eine in einer Frontalebene aufgenommene Schichtaufnahme 201 eines Stumpfes. In der Schichtaufnahme 201 sind der Femur (Oberschenkelknochen) 202, das Os coxae (Hüftbein) mit dem os ischii (Sitzbein) 204 und speziell der Ramus ossis ischii (Sitzbeinast) 203 erkennbar. Das in Schritt 110 (siehe Fig. 1) zu modifizierende 3D-Schaft-Stumpfmodell ist insbesondere so anzupassen, dass ein am Stumpf getragener Schaft einen festen Druck Druck auf den Ramus ossis ischii 203 und/oder Tuber ischiadicum 205 ausübt, um eine gute Kontrollierbarkeit und Steuerbarkeit der Prothese zu erreichen und keinen übermäßigen Druck auf das die Knochenstrukturen umliegende Gewebe ausübt, um die Durchblutung des Stumpfes beim Tragen des Schaftes nicht zu stören.

Ferner zeigt Fig. 2 einen Liner 206 auf, der strumpfartig über den Stumpf angezogen werden kann. Der aus Silikon oder anderen geeigneten Materialien bestehende Liner 206 liefert einerseits einen Dichteschwellwert bei der CT-Aufnahme, um umliegende nicht benötigte Strukturen, z.B. CT-Tisch, anderes (unversehrtes) Bein, Genitalien etc. leicht von dem Stumpfgewebe unterscheiden zu können. Andererseits dient der Liner 206 einer leichten Vorkompression, die einer möglichen Querformung des Stumpfes bei Lage des Patienten auf dem CT-Tisch entgegenwirkt.

Eine in Fig. 2 definierte Stumpfachse 207 führt durch einen Punkt des Articulatio coxae (Hüftgelenk) und beispielsweise durch einen Pukt des gedachten Articulatio genus (Kniegelenk) bzw. einen anderen Punkt gemäß der Motorik des Stumpfes.

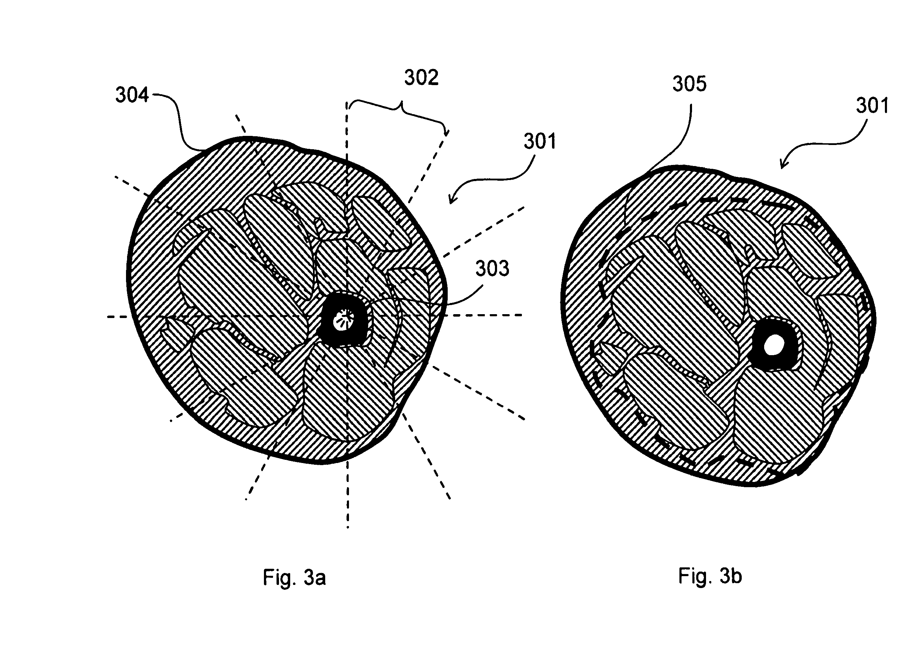

Fig. 3a zeigt eine ca. 1cm dicke Scheibe 301 segmentierter Gewebebereiche aus einem 3D-Schaft-Stumpfmodell. Die Scheibe 301 ist in zwölf gleiche Winkelsektoren 302 aufgeteilt, die sich ausgehend vom Zentrum des Femur 303 radial zur Oberfläche 304 des Stumpfes erstrecken. In den Winkelsegmenten 302 bildet beispielsweise ein Quotient aus den dort vorliegenden Teilvolumina von Fett und Muskulatur zusammen mit einer für jedes Gewebe bestimmten Kompressionszahl eine Ausgangsbasis, um die Gewebebereiche in den Winkelsektoren 302 im 3D-Schaft-Stumpfmodell individuell zu modifizieren. Außerdem können Erfahrungswerte aus Messreihen von Schaft-Probanden als weiterer Dimensionierungsfaktor für das Modifizieren einfließen. Alternativ kann beim Modifizieren lediglich die Kompressionszahl des Fettgewebes eingehen. Andere Gewebearten bleiben dann bei der Modifikation unberücksichtigt.

Insbesondere kann das Modifizieren des 3D-Schaft-Stumpfmodells in eine Anpassung eines distalen und eines proximalen Stumpfteils unterteilt werden. Dazu wird bevorzugt eine Referenzebene und eine Nullebene definiert, wobei die Referenzebene den distalen Teil des Tuber ischiadicum schneidet.

Das Anpassen eines distalen Stumpfteils beginnt dabei etwa 5cm unterhalb des Tuber ischiadicum 205, wo die Nullebene vorteilhafterweise definiert werden kann, in 1cm oder unterschiedlich dicken Scheiben, wie oben erläutert. Dies entspricht vor allem einem hydrostatischen Ansatz des distalen Schaftanteils. Das Anpassen des distalen Stumpfanteils endet bevorzugt in der Schicht, welche den distalen Endpunkt des Femurs 303 umfasst.

Das Anpassen des proximalen Stumpfteils erfolgt anhand wissensbasierter Regeln, die sich an der knöchernen Struktur des Femur 303, des Os coxae und wichtiger Hüftmuskeln orientieren. Zunächst erfolgt gemäß den wissensbasierten Regeln eine virtuelle eckige Freilegung der Sehne des Adductor longus, eine frontale flächige Anstützung des Muskulus rektor femoris, eine komplette Aussparung des Gluteus maximus sowie eine Vorspannung des Adductor magnus. Nach einer anschließenden Umgreifung des Tuber ischiadicum 205 folgt eine mediale Umgreifung des Ramus, eine flächige Umgreifung des Trochanter mit einer flächigen Anlage des Trochantermassivs.

Fig. 3b veranschaulicht ein Ergebnis einer individuellen Modifizierung der Gewebearten in den Winkelsektoren 302 der 3D-Schaft-Stumpfmodell-Scheiben 301 . Für Fig. 3b wurden kleinere Winkelsektoren 302, z.B. mit einem Mittelpunktswinkel oder Scheitelwinkel im Bereich von etwa 5 - 20° gewählt. Die neue Kontur 305 entspricht einer optimalen Anpassung des 3D-Schaft-Stumpfmodells an den Stumpf durch Reduzierung der Stirnseite der Winkelsektoren 302 anhand wissensbasierter Regeln.

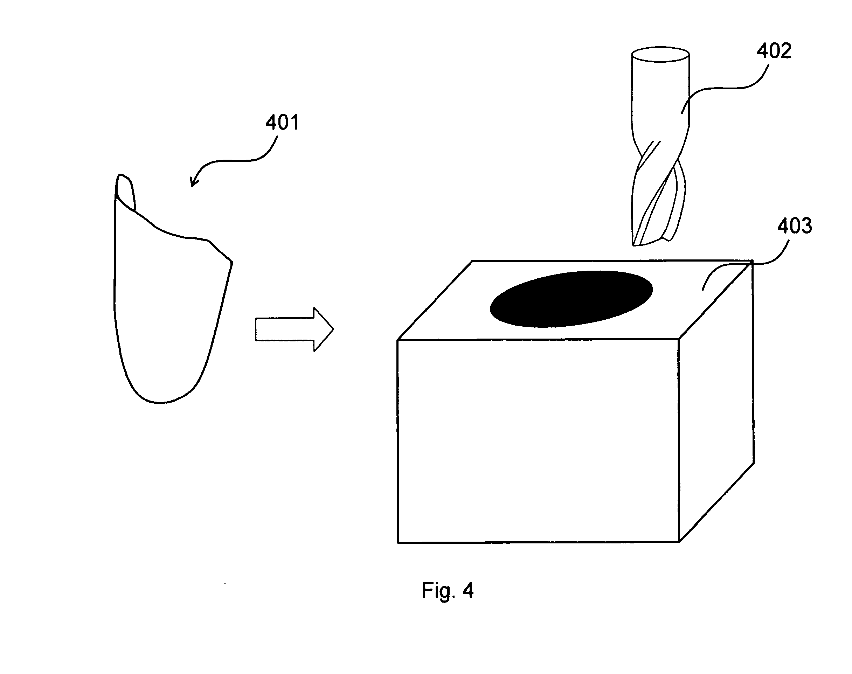

Fig. 4 zeigt ein nach Schritt 110 (siehe Fig. 1) generiertes 3D-Schaft-Stumpfmodell 401. In einem Zwischenschritt wird das 3D-Schaft-Stumpfmodell 401 in CAD-Daten für eine herkömmliche Fräsmaschinensteuerung umgesetzt. Ein Fräserwerkzeug 402 fräst dann den Schaft bzw. ein Stumpfpositiv aus einem Vollmaterial 403.

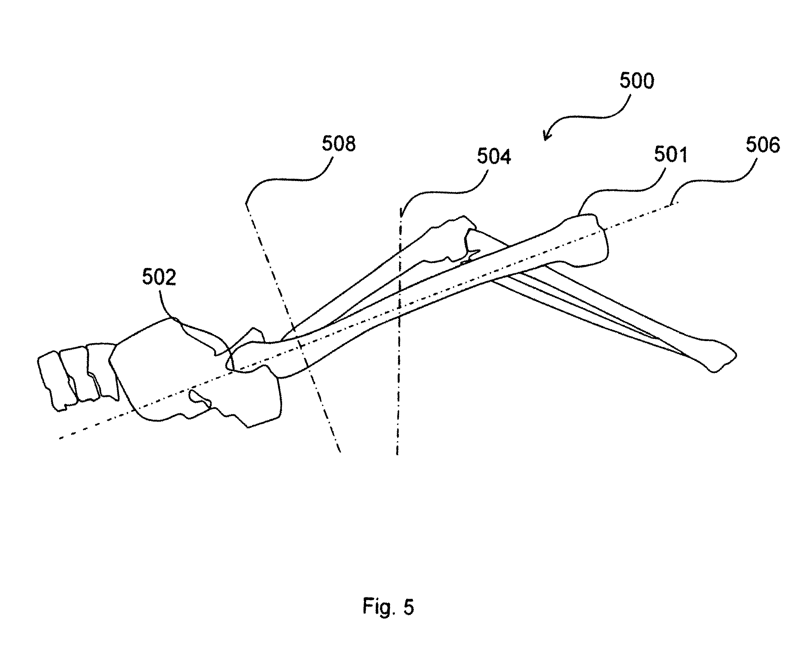

Fig. 5 zeigt Lagen von Achsen in einem 3D-Schaft-Stumpfmodell 500. Ein Stumpf bildender Körperteil mit einem Femur 501 liegt zu einem Zeitpunkt, an dem 3D-Bilddaten von ihm akquiriert werden, in Fig. 5 in nach oben abgewinkelter Stellung vor, d.h. der Femur 501 ist gebeugt. Ein CT liefert dabei konstruktionsbedingt Schnittbilder, die parallel zu Achse 504 ausgerichtet sind und welche die Symmetrie des Stumpfes nicht optimal berücksichtigen. Vorteilhafterweise wird bei der Rekonstruktion des 3D-Schaft-Stumpfmodells, z.B. Schritt 108, ein oberer Stumpfachsenpunkt an der Fossa acetabuli 502 festgelegt. Die Fossa acetabuli 502 liegt mittig des Articulatio coxae (Hüftgelenk), so dass der obere Stumpfachsenpunkt entsprechend der Motorik in deren Bewegungszentrum angeordnet ist. Ein unterer Stumpfachsenpunkt liegt bevorzugt in der geometrischen Mitte des distalen Endes des den Stumpf bildenden Körperteils. Der obere und der untere Stumpfachsenpunkt legen eine Stumpfachse 506 fest, die unabhängig von der verwendeten Aufnahmemodalität ist. Das 3D-Schaft-Stumpfmodell 500 ist senkrecht zur Stumpfachse 506 und parallel zu Achse 508 in Scheiben oder Bereiche unterteilbar. Die Stumpfachse 506 verläuft beispielweise in der Hauptrichtung von am Stumpf bildenden Körperteil auftretenden Kräften.

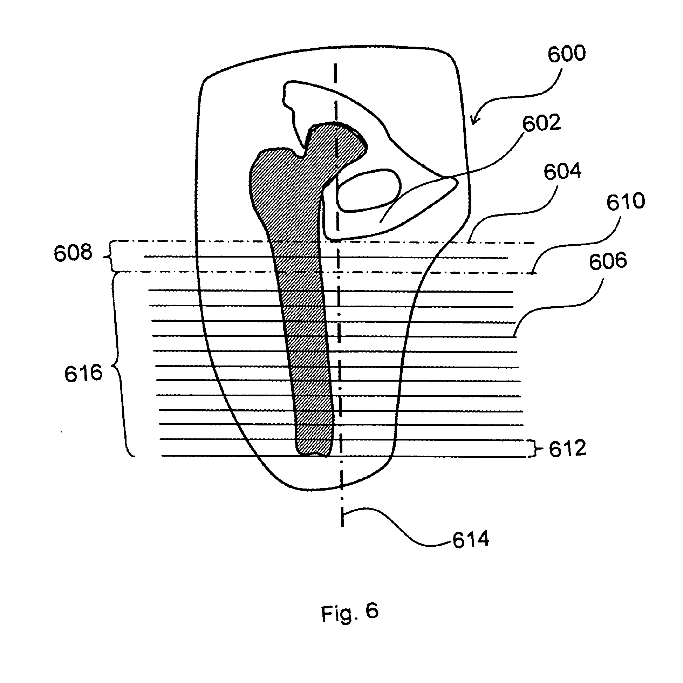

Fig. 6 zeigt ein 3D-Schaft-Stumpfmodell 600 und darin mehrere Scheiben bzw. Schnitte 604, 606, 610, die im Wesentlichen senkrecht zu einer Stumpfachse 614 angeordnet sind und das 3D-Schaft-Stumpfmodell 600 unterteilen. Die Scheibe 604 entspricht an ihrer Oberseite am distalen Ende des Tuber 602 der Referenzebene 604 und bildet eine Bezugsebene für die Bestimmung weiterer Scheiben oder Ebenen 604, 610, 606. Die Ebene 610 ist in einem vorbestimmten Abstand 608 von der Bezugsebene 604 entfernt, z.B. 5cm. Dabei handelt es sich um die Nullebene 610, welche den distalen 616 und den proximalen 608 des Stumpfes trennt. Daran schließen beliebig viele weitere distale Scheiben 606 an. Die Dicke 616 der Scheiben ist beliebig. Es kann von Vorteil sein, dass die Dicke 612 der Scheiben 606 zum distalen Ende des 3D-Schaft-Stumpfmodells 600 hin zunimmt. Die Dicke der Scheiben 606, 608 kann invers zur Komplexität der in den Scheiben 606, 608 enthaltenen Gewebebereiche und/oder Anatomie und/oder der Verteilung von Gewebearten sein. Nahe dem Tuber 602 kann durch eine geringere Dicke 612 der Scheiben 604 eine genauere Anpassung des 3D-Schaft-Stumpfmodells 600 an den Stumpf ermöglicht werden. Alternativ kann bei Anpassung im proximalen Bereich 608 eine größere Scheibendicke 608 vorteilhaft sein, wenn das 3D-Schaft-Stumpfmodell mittels als Absolutwert angegebenen Volumenkompressionsfaktoren an wesentliche Knochenstrukturen angepasst werden soll.

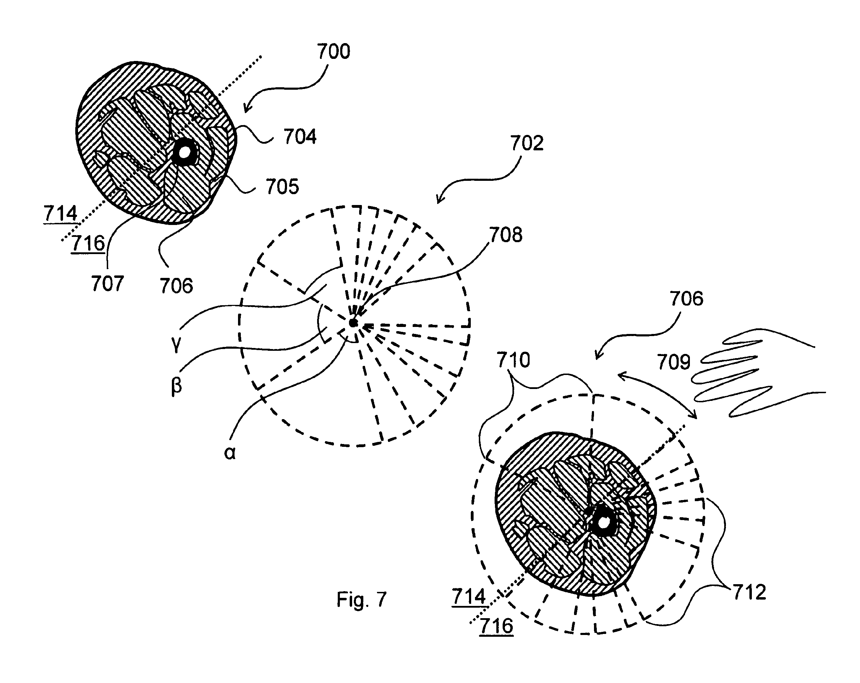

Fig. 7 zeigt eine Scheibe 700 mit Winkelsektoren 702. Da verschiedene Scheiben 700 je nach ihrer Position im 3D-Schaft-Stumpfmodell 600 unterschiedliche Verteilungen der Gewebearten, z.B. Haut 704, Fett 705, Muskeln 706, und Knochen 707, aufweisen können, wird die Scheibe 700 in eine bestimmte Anzahl an Winkelsektoren 702 unterteilt. Die Winkelsektoren 702 können, wie in Fig. 7 gezeigt verschiedene Winkelanteile oder Mittelpunktswinkel α, β, γ aufweisen. Bevorzugt sind die Mittelpunktswinkel α, β, γ medial 716 kleiner als lateral 714. Dies ist besonders sinnvoll, wenn beispielsweise im proximalen Bereich des Stumpfes an wesentliche Knochenstrukturen angepasst werden soll. So enthalten die medial liegenden Winkelsektoren 712 alle einen Teil des Femurknochens 707, welcher nicht komprimierbar ist. Um hier eine exakte Anpassung zu ermöglichen, sind die Winkelsektoren 712 klein gewählt. Im Gegensatz dazu weisen die Winkelsektoren im lateralen Bereich 710 große Anteile Fett auf. Der Mittelpunkt 706 der Winkelsektoren 702 wird in Übereinstimmung mit der Stumpfachse, z.B. 207, 506, 614, gebracht.

Mittels Rotation 709, beispielsweise durch den Benutzer, der Winkelsektoren 702 um die Stumpfachse kann eine optimale Überlagerung der Gewebeverteilungen im 3D-Schaft-Stumpfmodell mit den Winkelsektoren 702 in jeder Scheibe 700, 301, 606, 608 erreicht werden.

Zum Beispiel würden die Sektoren 710 der Scheibe 700 eine höhere Komprimierung erlauben, da sie mehr komprimierbare Gewebearten, wie Muskeln 706 und Fett 705 enthalten als die Sektoren 712. Die Sektoren 712 enthalten wenig komprimierbares Fett 705 und einen Abschnitt des nicht komprimierbaren Femurs 707.

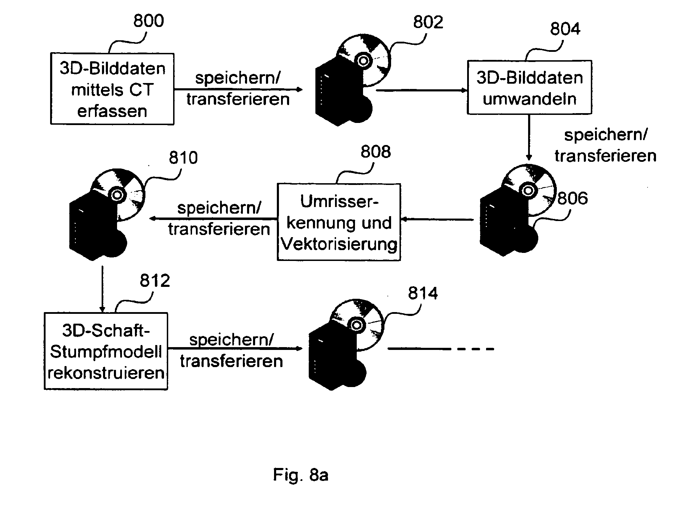

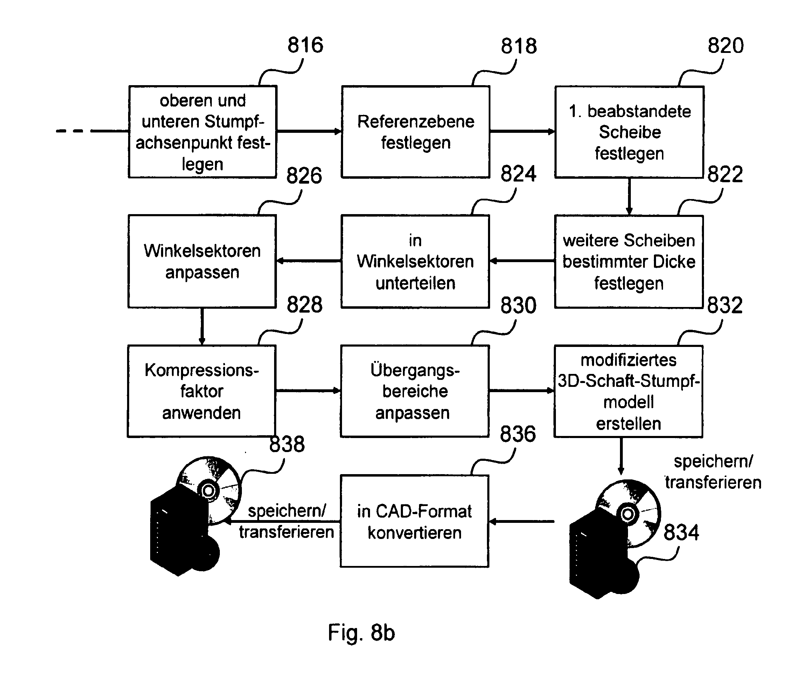

Fig. 8a und 8b zeigen ein Ablaufdiagramm zur Erstellung eines 3D-Schaft-Stumpfmodels. Zunächst werden in Schritt 800 3D-Bilddaten mittels CT erfasst und anschließend in einem Speicherschritt 802 auf einen Server transferiert und/oder dort gespeichert oder auf einem Speichermedium gespeichert, um transportabel zu sein. Im folgenden Schritt 804 werden die 3D-Bilddaten aus Schritt 800 in ein anderes Speicherformat umgewandelt; danach folgt ein weiterer Speicherschritt 806. Ein Schritt 808 zur Umrisserkennung und Vektorisierung der umgewandelten 3D-Bilddaten erzeugt segmentierte 3D-Bilddaten, die wiederum einem Speicherschritt 810 unterzogen werden. Im folgenden Schritt 812 wird aus den segmentierten 3D-Bilddaten ein 3D-Schaft-Stumpfmodell rekonstruiert und wieder einem Speicherschritt 814 unterzogen. In Schritt 816 wird ein oberer und ein unterer Stumpfachsenpunkt festgelegt, die gemeinsam eine Stumpfachse definieren können. Im folgenden Schritt 818 wird eine Referenzebene festgelegt. Danach wird in einem vorbestimmten Abstand zur Referenzebene eine Nullebene festgelegt. Im danach folgenden Schritt 822 werden weitere Scheiben, z.B. Scheiben 606, festgelegt, die jeweils eine bestimmte Dicke, z.B. Dicke 612, aufweisen. Im Folgenden Schritt 824 wird eine Scheibe in Winkelsektoren unterteilt, wobei die Winkelsektoren in Schritt 826 an die Verteilung der Gewebearten in der jeweiligen Scheibe angepasst werden können. In Schritt 828 werden Kompressionsfaktoren auf die einzelnen Winkelsektoren der jeweiligen Scheibe angewendet. Dabei wird bevorzugt die außen liegende Fläche eines Winkelsektors beibehalten und lediglich verschoben. Wie weit die Außenfläche verschoben wird, wird anhand der in dem Winkelsektor vorhandenen Kompressionswerte ermittelt. Liegt beispielsweise nur ein Kompressionswert für Fettgewebe in einem Winkelsektor vor, so wir die Kompression des Fettgewebes in einen resultierenden Kompressionsfaktor für den Winkelsektor überführt, der die verschiedenen Gewebeanteile in dem Winkelsektor berücksichtigt. Liegen Kompressionswerte für beispielsweise Fettgewebe und Muskelgewebe vor, so wird anhand dieser beiden Kompressionswerte und der vorliegenden Gewebeverteilung ein resultierender Kompressionswert für den Winkelsektor ermittelt. Daraus kann die Strecke, um welche die Außenfläche radial verschoben werden muss, berechnet werden. Durch die Kompression der Scheiben können stufenförmige Übergangsbereiche zwischen benachbarten Scheiben auftreten. Im folgenden Schritt 830 werden diese Übergangsbereiche angepasst oder geglättet. Danach wird in Schritt 832 ein modifiziertes 3D-Schaft-Stumpfmodell erstellt und einem Speicherschritt 834 unterzogen. Schließlich erfolgt in Schritt 836 eine Konvertierung des 3D-Schaft-Stumpfmodells in ein CAD-Format sowie ein letzter Speicherschritt 838. Eine Fräsmaschine kann somit das 3D-Schaft-Stumpfmodell im CAD-Format zur Herstellung eines Prothesenschafts verwenden.

Die Schritte 824 bis 830 können für die jeweils vorgesehene Anzahl an Scheiben iteriert werden. In Schritt 824 können dafür mehrere vordefinierte Winkelsektoren 702 mit jeweils vorbestimmten Winkelanteilen α, β, γ verwendet werden, die in Schritt 826 nur noch an die Verteilung der Gewebearten, z.B. durch Rotieren der Winkelsektoren 702, angepasst werden. Gleiches gilt für das Anwenden von Kompressionsfaktoren in Schritt 828. Diese können manuell durch den Benutzer verändert werden. Das Anpassen der Übergangsbereiche in Schritt 830 kann bei jeder Iteration oder erst am Schluss an allen Übergangsbereichen erfolgen. Ferner können Schritte ausgetauscht und mit anderen kombiniert werden, z.B. Festlege-Schritte. Außerdem können Schritte entfallen, z.B. Speicherschritte, Umwandel- oder Konvertierschritte.

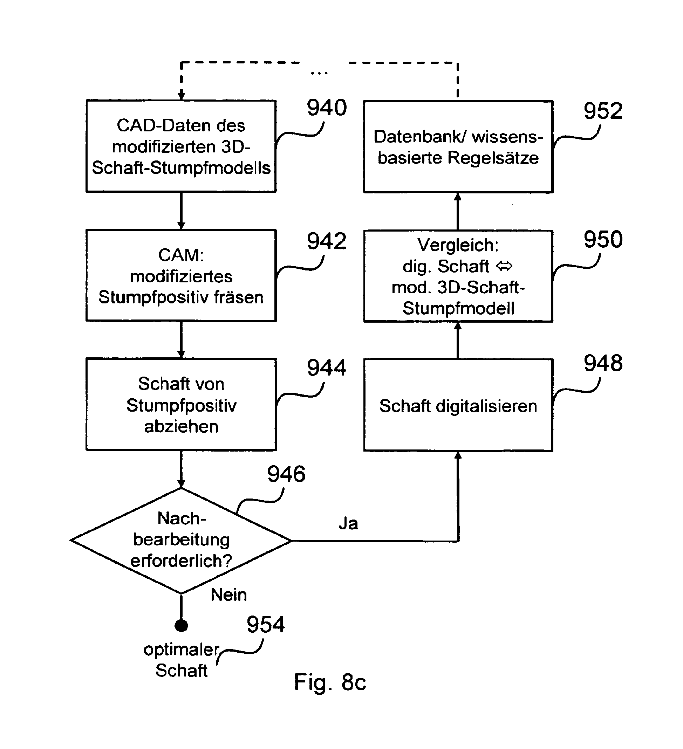

Fig. 8c zeigt ein weiteres Ablaufdiagramm zur Überarbeitung und/oder besseren Anpassung eines Schaftes. Dabei schließen die gezeigten Schritte in Fig. 8c direkt an Schritt 838 in Fig. 8c an. Zunächst werden in Schritt 840 die in ein CAD-Format konvertierten Daten des modifizierten 3D-Schaft-Stumpfmodells an eine Fräsmaschine übertragen, welche in Schritt 842 anhand der CAD-Daten ein StumpfPositiv des modifizierten 3D-Schaft-Stumpfmodells 401 erzeugt (vgl. Fig. 4). Mittels verschiedener Verfahren und Materialien kann nun in Schritt 844 ein Schaft aus dem Stumpf-Positiv hergestellt werden. Bevorzugt handelt es sich dabei um einen Karbon-Schaft oder einen Glasfaser-Schaft. Die Schaftform entspricht dabei dem Negativ des Stumpf-Positivs. In einem weiteren Schritt 846 kann nun eine Entscheidung erfolgen, ob der erstellte Schaft weiter bearbeitet werden muss. Diese Entscheidung kann nach einer einmaligen Anprobe des Schaftes durch den Patienten oder aber nach einem längeren Zeitraum des Schafttragens durch den Patienten erfolgen. Hat der Patient keine Beschwerden beim Tragen des Schaftes, so kann von einer weiteren Bearbeitung abgesehen werden und das Verfahren hat in einem einzigen Durchlauf einen optimalen Schaft 854 erstellt. Sind jedoch nach einer Untersuchung weitere Anpassungen nötig, so kann in einem nächsten Schritt 848 die Form des erstellten Schaftes digitalisiert werden. Das Digitalisieren erfolgt bevorzugt durch optische Abtastverfahren, beispielsweise mittels eines Lasers. In einem nächsten Schritt 850 kann mittels der erhaltenen Forminformationen über den erstellten Schaft zunächst ein Vergleich mit den Forminformationen des gewünschten, zu erstellenden Schaftes verglichen werden, um Fertigungsfehler ausschließen zu können.

Weiter kann es vorteilhaft sein, bei der Erstellung eines Schaftes die regelmäßig im zeitlichen Verlauf des Tragens eines Schaftes zu erwartenden typischen Veränderungen der Gewebeverteilungen oder Gewebevolumenveränderungen eines Schaftes zu berücksichtigen. So bildet sich beispielsweise das Muskelgewebe kurz nach der Amputation zurück. Diese typischen Veränderungen können über das Vermessen einer Vielzahl von Stümpfen von Patienten ermittelt werden. Die statistisch signifkanten typischen Gewebeveränderungen können in einer Datenbank abgelegt werden und bei der Modifizierung eines 3D-Schaft-Stumpf-Computermodells heran gezogen werden. Anhand dieser zusätzlich berücksichtigten Informationen kann eine "Sollform" des Schaftes ermittelt werden. Dabei umfassen die in einer Datenbank abgelegten Informationen Informationen über die Entwicklung der Form eines Stumpfes über die Zeit von einer Vielzahl von Patienten. Diese können statistisch ausgewertet werden, sodass sich eine signifikante mittlere Formveränderung ergibt, die zur Erstellung eines "Prognoseschaftes" heran gezogen werden kann. Vorteilhaft ist ein Prognoseschaft dann, wenn er die zu erwartenden Formveränderungen des Schaftes automatisch berücksichtigt, sodass sich die Zeitabstände, nach denen eine Schaftanpassung erforderlich ist, vergrößern. Die in der Datenbank abgelegten Forminformationen 952 für eine Vielzahl von Patienten können als Parameter von den wissensbasierten Regelsätzen berücksichtigt und ausgewertet werden und zu einer Veränderung der zu Grunde liegenden wissensbasierten Regeln führen.

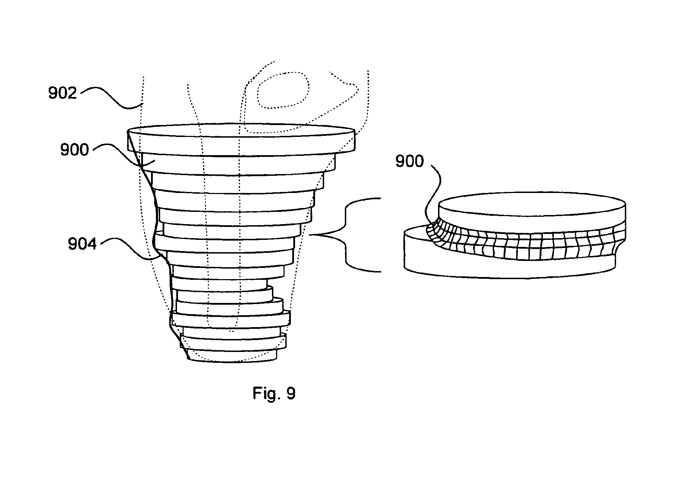

Fig. 9 zeigt schematisch den Glättungsprozess zwischen verschiedenen komprimierten Bereichen des 3D-Schaft-Stumpfmodells. Die komprimierten, übereinanderliegenden Schichten 900 bilden das komprimierte 3D-Schaft-Stumpfmodell. Die Außenflächen der einzelnen komprimierten Schichten 900 entsprechen der Konturlinie 305 in Fig. 3b. Zur Veranschaulichung wurde in gestrichelten Linien die unkomprimierte Stumpfform 902 überlagert. Entsprechend einer globalen Glättung, dargestellt durch die Flächenkontur 904, werden lokale Unebenheiten, die sich aus den einzelnen komprimierten Schichten ergeben, kompensiert, sodass eine homogene Modelloberfläche resultiert. Alternativ kann bevorzugt eine lokale Glättung 906 der Modelloberfläche durchgeführt werden. Dabei werden die Übergangsbereiche benachbarter Schichten 900 aneinander angeglichen, sodass insgesamt eine raue, inhomogene Modelloberfläche entsteht. Dies kann vorteilhafte Trageeigenschaften bewirken.

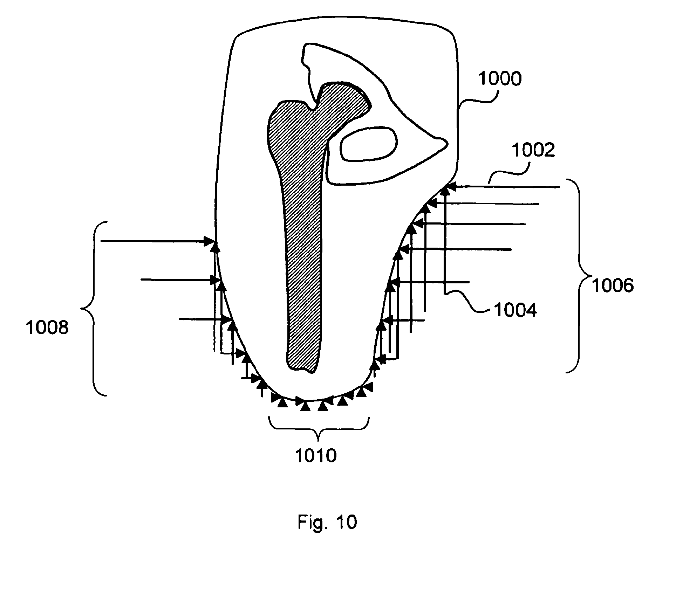

Fig. 10 zeigt die den auf das modifizierte 3D-Schaft-Stumpfmodell angewendeten Veränderungen oder Kompressionen entsprechende Krafteinwirkung auf den Stumpf bei Anliegen des zu erstellenden Schaftes. Dabei stehen die Pfeile 1008 für eine laterale Kraftverteilung auf den Stumpf, die Pfeile 1006 für eine mediale Kraftverteilung auf den Stumpf und die Pfeile 1010 für eine distale Kraftverteilung. Typischerweise ist die Kraftverteilung distal gleich oder annähernd 0. Weiter unterscheidet die Fig. 10 vertikale Kraftkomponenten, wie beispielsweise Pfeil 1004, welche im Wesentlichen parallel zur Stumpfachse wirken und horizontalen Kraftkomponenten, wie beispielsweise Pfeil 1002, welche im Wesentlichen senkrecht zur Stumpfachse wirken. Die resultierende Krafteinwirkung ergibt sich als Summe aller Kraftkomponenten. Entsprechend einer optimalen Druckverteilung und/oder Kraftverteilung nimmt die Krafteinwirkung global von proximal nach distal ab. Unter Berücksichtigung weiterer Parameter, wie bspw. des Körpergewichts des Patienten, kann das erfindungsgemäße Verfahren eine optimale Längskompression des 3D-Schaft-Stumpfmodells ermitteln.

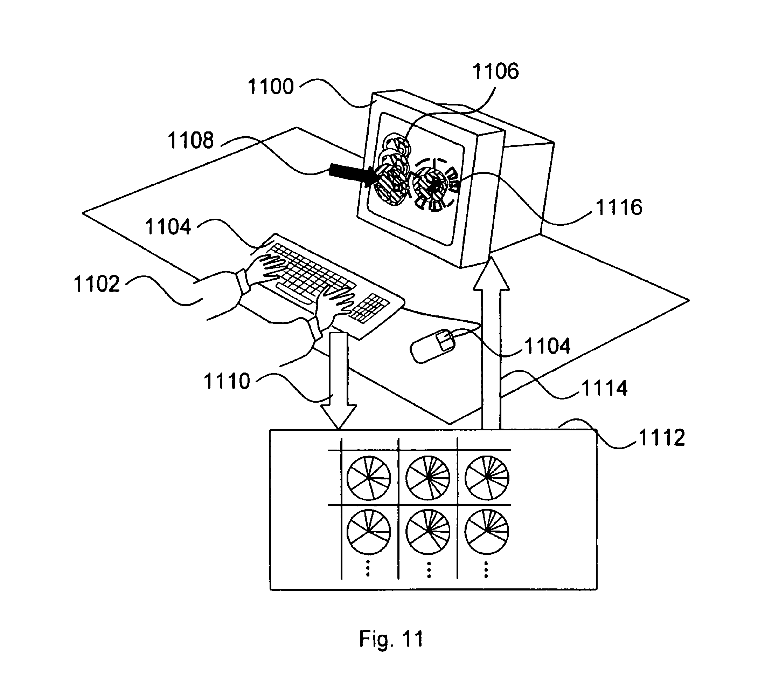

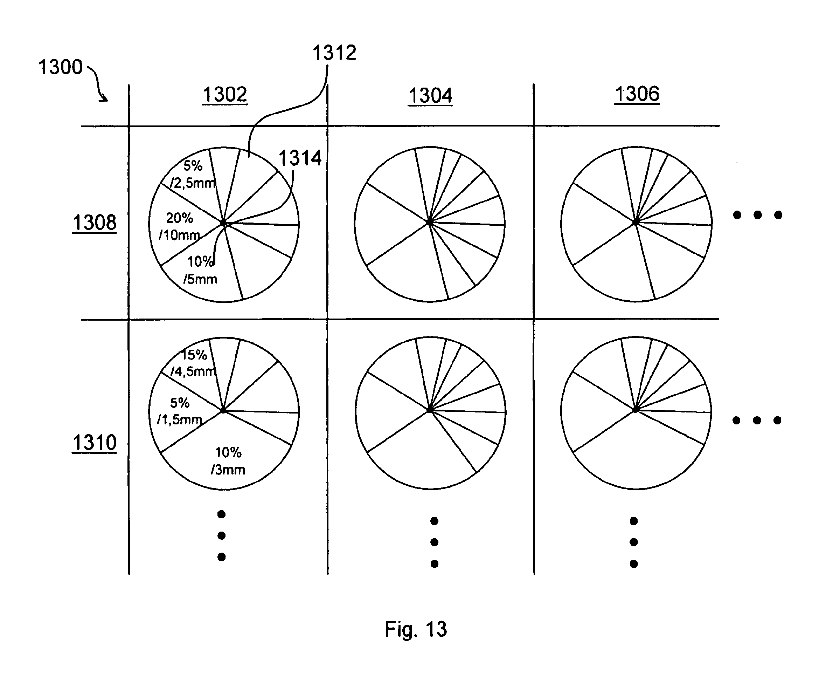

Fig. 11 zeigt die Interaktion eines Benutzers 1102 mit einem 3D-Schaft-Stumpf-Computermodell mittels wenigstens eines Interaktionsmoduls 1100, 1104, beispielsweise einem Computer-Monitor, einer Maus oder Tastatur zum Anzeigen von Informationen und zum Tätigen von Eingaben. In einem ersten Schritt der Interaktion 1108 kann der Benutzer über die Interaktionsschnittstelle 1104 einen Abschnitt 1106 des 3D-Schaft-Stumpf-Computermodells auswählen und über den Computerbildschirm zur Anzeige bringen. Die Anzeige erfolgt dabei derart, dass dem Benutzer die verschiedenen segmentierten Gewebearten in dem Abschnitt angezeigt werden. In einem zweiten Schritt der Interaktion 1116 wählt der Benutzer über ein Auswahlmodul 1112, beispielsweise dem Computer mit darin in einer Datenbank abgelegten Templates und/oder räumlichen Verteilungen, wenigstens eine räumliche Verteilung einer Volumenänderung für den angezeigten Abschnitt aus. Eine räumliche Verteilung ist insbesondere eine Unterteilung des Abschnittes in Winkelsektoren. Dazu kann der Benutzer z.B. mittels einer Eingabe 1110 über die Schnittstelle 1104 aus einer Tabelle von Templates (siehe hierzu Fig. 13), die ihm über die Interaktionsschnittstelle 1100 angezeigt wird, eines auswählen. Die Eingabe 1110 wird durch das Auswahlmodul 1112 verarbeitet und in eine Ausgabe 1114 überführt, die einem Befehl entspricht, das ausgewählte Template 1116 mit dem Abschnitt 1106 zu überlagern. Die Überlagerung von Template und Abschnitt kann dem Benutzer angezeigt werden. Zusammen mit dem Template wählt der Benutzer auch Kompressionsfaktoren für einen jeden Unterbereich des Templates aus. In einem weiteren Schritt kann der Benutzer einen Kompressionswert eines Unterbereiches per Eingabe nachträglich verändern und besser an die Gewebeverteilung im Abschnitt anpassen. Auch ist es vorgesehen, dass der Benutzer die Unterbereiche eines Templates, z.B. durch Rotieren an die Gewebeverteilung besser anpasst. Durch Bestätigung durch den Benutzer werden die wissensbasierten Regelsätze auf den Abschnitt angewendet, was zu einer Volumenkompression des Abschnittes führt (siehe Fig. 3). Der komprimierte Abschnitt kann ebenfalls dem Benutzer angezeigt werden, sodass dieser eine Möglichkeit hat, die resultierende Kompression zu überprüfen. Ist der Benutzer mit dem Resultat nicht zufrieden, kann er erneut einen Optimierungsschritt durchführen, indem er das Template erneut rotiert und den Kompressionsschritt mit der angepassten Lage des Templates relativ zum Abschnitt wiederholt und oder erneut wenigstens einen der Volumenkompressionsfaktoren in einem der Winkelsektoren verändert. Akzeptiert der Benutzer das Resultat der Kompression, so kann er in einem weiteren Schritt einen weiteren Abschnitt des 3D-Schaft-Stumpfmodells auswählen und diesen wie beschrieben modifizieren.

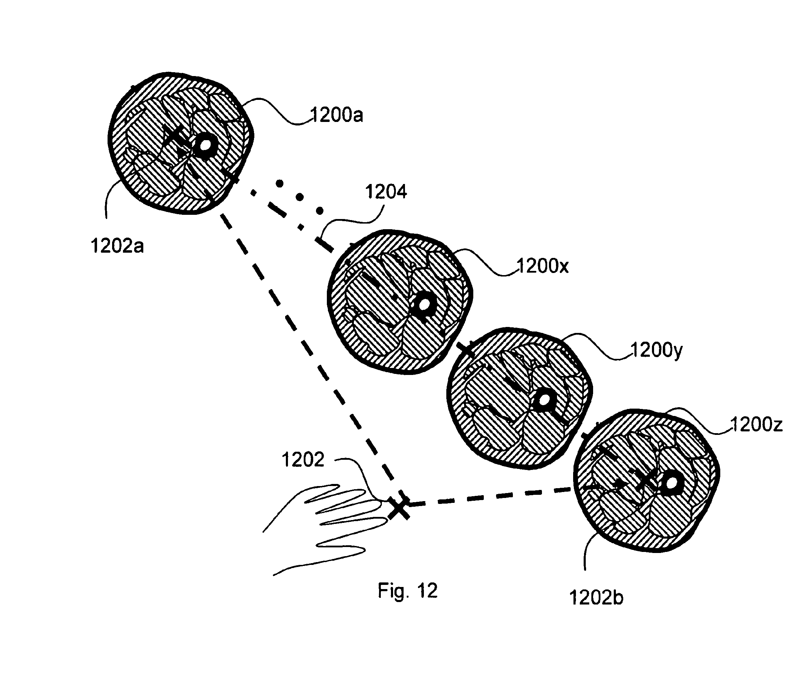

Fig. 12 zeigt die Interaktion eines Benutzers (dargestellt durch die Hand) mit einem 3D-Schaft-Stumpf-Computermodell zum Festlegen einer Stumpfachse in dem 3D-Schaft-Stumpf-Computermodell. Dabei wählt der Benutzer zunächst ein Schnittbild 1200x, 1200y oder 1200z, welches mittels eines medizinischen Bildgebungsverfahrens, z.B. MRT oder CT aufgenommen wurde aus, welches einen Ausschnitt des distalen Bereiches des 3D-Schaft-Stumpf-Computermodells darstellt. Der Benutzer verwendet dieses Schnittbild, um einen distalen Punkt 1202b, durch den die Stumpfachse verlaufen soll, zu bestimmen. Weiter kann der Benutzer sich das ausgewählte distale Schnittbild anzeigen lassen und beispielsweise über die Interaktionsschnittstellen einen auf dem Display des Computermonitors frei beweglichen Punkt 1202 auf der gewünschten Position in dem ausgewählten Schnittbild platzieren. Vorzugsweise legt der Benutzer den Punkt im geometrischen Mittelpunkt des Schnittbildes oder durch das Zentrum des Femurs fest. Eine analoge Schrittfolge wird beim Festlegen eines proximalen Punktes 1202a durchlaufen, welcher sich in einem proximalen Abschnitt 1200a befindet. Hier wird der proximale Punkt der Schnittachse bevorzugt an Knochen- oder Knorpelstrukturen des Hüftgelenkes bestimmt. Anschließend wird eine Stumpfachse berechnet, die beide festgelegten Punkte schneidet. Anhand der festgelegten Stumpfachse kann dann das 3D-Schaft-Stumpfmodell in Scheiben bestimmter Dicke unterteilt werden, die im Wesentlichen senkrecht zu der Stumpfachse angeordnet sind.

Fig. 13 zeigt einen Ausschnitt einer Template-Bibliothek 1300. Die Template-Bibliothek ist bevorzugt in einer Datenbank in einem Datenbankmodul hinterlegt, auf welche das zweite Auswahlmodul zugreifen kann. Die Template-Bibliothek umfasst eine Vielzahl von räumlichen Verteilungen der Modifizierung 1312 für Scheiben in einem 3D-Schaft-Stumpf-Computermodell und die dazu gehörigen Kompressionsfaktoren 1314, beispielsweise kann es sich bei einer räumlichen Unterteilung um eine Winkelsektor-Unterteilung handeln. Jedem Winkelsektor wird dabei ein Kompressionswert oder mehrere Kompressionswerte für verschiedene, in dem Unterbereich enthaltene Gewebearten zugeordnet. Die Kompressionsfaktoren 1314 werden als Prozentwert angegeben oder entsprechen einer absoluten Volumenänderung. Beispielsweise kann ein Kompressionswert eine Volumenänderung von 5% angeben. Alternativ kann ein Kompressionswert auch 5mm betragen. Dann wird der die die Außenfläche des 3D-Schaft-Stumpf-Computermodell bildende Seite des Winkelsektors um diesen Betrag radial verschoben, was in einer entsprechenden Volumenänderung resultiert. In der Datenbank 1300 werden die räumlichen Verteilungen verschiedenen Kategorien 1302, 1304, 1306, 1308, 1310 zugeordnet, was eine Verwaltung der räumlichen Verteilungen erleichtert. Die Kategorien werden bevorzugt anhand der Lage eines ausgewählten Abschnittes innerhalb des Stumpfes festgelegt. So findet man räumliche Verteilungen in der Kategorie proximal 1308 und distal 1310. Ferner können die Kategorien den physiologischen oder anatomischen Voraussetzungen eines Patientenstumpfes und/oder des Patienten selbst und/oder der Gewebeverteilung im dem einen Abschnitt Rechnung tragen. So unterscheiden sich die räumlichen Verteilungen beispielsweise für einen männlichen und einen weiblichen Patienten oder für einen linken oder rechten Stumpf. Auch ist bei der Auswahl eines Templates zu berücksichtigen, ob es sich um einen langen oder um einen kurzen Stumpf handelt. Bei der Kategorie 1302 kann es sich beispielsweise um räumliche Verteilungen für linksseitige lange Stümpfe männlicher Patienten handeln, die insgesamt einen hohen Muskelanteil aufweisen, wohingegen die Kategorie 1304 räumliche Verteilungen für kurze Stümpfe weiblicher Patienten mit höherem Fettgehalt beinhaltet. Die Liste der Kategorien ist nicht abschließend. Die Erstellung und/oder Anpassung der räumlichen Verteilungen und/oder Templates in der Datenbank kann manuell durch einen Benutzer oder automatisch über Selbstlernprozesse unter Auswertung von beispielsweise vorherigen Modifizierungen eines 3D-Schaft-Stumpf-Computermodells erfolgen. Ein Anpassen der räumlichen Verteilungen in der Datenbank 1300 kann ein Entfernen, ein Hinzufügen und/oder ein Verändern von einzelnen räumlichen Verteilungen 1312 umfassen. Bei der Anpassung einzelner räumlicher Verteilungen kann die Winkelsektor-Unterteilung angepasst und/oder deren Kompressionswerte verändert werden. Weiter kann das Anpassen der räumlichen Verteilungen auch das Löschen oder Erstellen ganzer Kategorien von räumlichen Verteilungen umfassen.

## Patentansprüche

1. Vorrichtung zur Interaktion (1110) eines Benutzers (1102) mit einem 3D-Schaft-Stumpf-Computermodell zur Modifizierung des 3D-Schaft-Stumpf-Computermodells (109; 401; 500; 600), das die Oberflächenform und räumliche Gewebeverteilung eines Stumpfes beschreibt, wobei die Vorrichtung ausgelegt ist, das 3D-Schaft-Stumpf-Computermodell (109; 401; 500; 600) in Abschnitte (301; 700; 900; 1106) zu unterteilen, mit:
- einem Display (1100), welches ausgelegt ist, die Oberflächenform und Gewebeverteilung in einem Abschnitt (301; 700; 900; 1106) des 3D-Schaft-Stumpf-Computermodells (109; 401; 500; 600) anzuzeigen; und
- einem ersten Auswahlmodul, welches dem Benutzer (1102) ermöglicht, einen Abschnitt (301; 700; 900; 1106) des 3D-Schaft-Stumpf-Computermodells (109; 401; 500; 600) zur Anzeige an dem Display (1100) auszuwählen (1108); **gekennzeichnet durch**
- ein zweites Auswahlmodul (1112), das dem Benutzer (1102) ermöglicht, wenigstens eine vorab festgelegte räumliche Verteilung einer Modifizierbarkeit der Oberflächenform in dem Abschnitt (301; 700; 900; 1106) auszuwählen;
wobei die Vorrichtung ausgelegt ist, entsprechend der ausgewählten räumlichen Verteilung die Oberflächenform in dem Abschnitt (301; 700; 900; 1106) zu modifizieren (110; 828; 1116).

2. Vorrichtung gemäß Anspruch 1, wobei die Vorrichtung ausgelegt ist, dem Benutzer (1102) zu ermöglichen, eine Stumpfachse (207; 614; 1204) anhand des 3D-Schaft-Stumpf-Computermodells (109; 401; 500; 600) festzulegen und ausgelegt ist, das 3D-Schaft-Stumpf-Computermodell (109; 401; 500; 600) in Abschnitte (301; 700; 900; 1106) zu unterteilen, welche an der Stumpfachse (207; 614; 1204) ausgerichtet sind.

3. Vorrichtung gemäß Anspruch 2, wobei die Abschnitte (301; 700; 900; 1106) im Wesentlichen senkrecht zur Stumpfachse (207; 614; 1204) liegende Scheiben (301; 700; 900; 1106) sind und die Vorrichtung ausgelegt ist, dem Benutzer (1102) zu ermöglichen, die Dicke einer Scheibe (301; 700; 900; 1106) individuell zu bestimmen.

4. Vorrichtung gemäß einem der vorhergehenden Ansprüche, wobei die vorab festgelegte räumliche Verteilung eine Unterteilung des Abschnittes (301; 700; 900; 1106) in wenigstens einen Unterbereich (302; 702; 1312) umfasst, wobei jedem Unterbereich (302; 702; 1312) wenigstens ein Wert (1314) zugeordnet ist, auf dem die Stärke der Modifizierung der Oberflächenform basiert.

5. Vorrichtung gemäß Anspruch 4, die ausgelegt ist, dass sie auf Basis des Wertes (1314) und der Gewebeverteilung in dem Unterbereich (302; 702; 1312) eine Volumenänderung einer der in dem Unterbereich (302; 702; 1312) enthaltenen Gewebearten ableitet und anhand der Volumenänderung die Stärke der Modifizierung der Oberflächenform bestimmt.

6. Vorrichtung gemäß Anspruch 5, wobei die Gewebearten Fett, Muskel, Haut und/oder Knochen umfassen und der Wert (1314) die prozentuale Volumenänderung der jeweiligen Gewebeart (104-107; 704-707) angibt und auf der Kompressibilität der jeweiligen Gewebeart basiert.

7. Vorrichtung gemäß den Ansprüchen 4 bis 6, bei der das zweite Auswahlmodul (1112) dem Benutzer (1102) ermöglicht, den Wert (1314) manuell zu verändern.

8. Vorrichtung gemäß den Ansprüchen 4 bis 6, wobei der Unterbereich (302; 702; 1312) ein Winkelsektor (302; 702; 1312) ist, der radial ausgehend von der Stumpfachse (207; 614; 1204) angeordnet ist.

9. Vorrichtung gemäß Anspruch 7 oder 8, bei der es das zweite Auswahlmodul (1112) dem Benutzer (1102) ermöglicht, die räumliche Ausrichtung des Winkelsektors (302; 702; 1312) durch Drehen (709) um die Stumpfachse (207; 614; 1204) manuell zu verändern.

10. Vorrichtung gemäß einem der vorhergehenden Ansprüche, derart ausgestaltet, dass bei der Modifizierung (110; 828; 1116) die Form der die Außenfläche des 3D-Schaft-Stumpf-Computermodells (109; 401; 500; 600) bildenden Fläche des Unterbereiches (302; 702; 1312) im Wesentlichen erhalten bleibt.

11. Vorrichtung gemäß einem der vorhergehenden Ansprüche, bei der das zweite Auswahlmodul (1112) derart ausgelegt ist, dass die vorab festgelegten räumlichen Verteilungen, aus denen der Benutzer (1102) für den jeweiligen Abschnitt (301; 700; 900; 1106) auswählen kann, abhängen von:
- der Lage des Abschnittes (301; 700; 900; 1106) innerhalb des Stumpfes;
- der Gewebeverteilung des Abschnittes (301; 700; 900; 1106); und/oder
- physiologischen oder anatomischen Eigenschaften des Stumpfes bzw. des Patienten.

12. Vorrichtung gemäß einem der vorhergehenden Ansprüche, die auch ein Datenbankmodul umfasst, in dem die vorab festgelegten räumlichen Verteilungen abgelegt sind und die Vorrichtung ausgelegt ist, basierend auf einer Analyse von vorausgegangenen Modifizierungen (110; 828; 1116) des 3D-Schaft-Stumpf-Computermodells (109; 401; 500; 600) anderer und/oder desselben Patienten den Satz der räumlichen Verteilungen in der Datenbank anzupassen.

13. Vorrichtung gemäß einem der vorhergehenden Ansprüche, bei der das zweite Auswahlmodul (1112) derart ausgelegt ist, dass die vorab festgelegten räumlichen Verteilungen, aus denen der Benutzer (1102) für den jeweiligen Abschnitt (301; 700; 900; 1106) auswählen kann, eine physiologisch zu erwartende Veränderung des Stumpfes berücksichtigen.

14. System zur Interaktion (1110) eines Benutzers (1102) mit einem 3D-Schaft-Stumpf-Computermodell (109; 401; 500; 600) zur Modifizierung des 3D-Schaft-Stumpf-Computermodells (109; 401; 500; 600), wobei das System umfasst:
a) eine Einleseeinheit zum Einlesen von 3D-Bilddaten des Stumpfes;
b) eine Segmentiereinheit (103) zum Segmentieren der 3D-Bilddaten zur Bestimmung der räumlichen Gewebeverteilung des Stumpfs;
c) eine Rekonstruiereinheit (108) zum Rekonstruieren eines 3D-Schaft-Stumpf-Computermodells (109; 401; 500; 600) anhand der segmentierten 3D-Bilddaten, welches Oberflächenform und räumliche Gewebeverteilung des Stumpfes beschreibt;
d) eine Vorrichtung zur Interaktion (1110) mit einem Benutzer (1102) nach einem der Ansprüche 1 bis 13, welche das 3D-Schaft-Stumpf-Computermodell (109; 401; 500; 600) unter Berücksichtigung von Eingaben des Benutzers (1102) modifiziert; und
e) eine Ausgabeeinheit, welche das modifizierte 3D-Schaft-Stumpf-Computermodell (109; 401; 500; 600) zur Weiterverwendung für die Herstellung eines Prothesenschaftes ausgibt.

15. Verfahren zur Erstellung eines 3D-Schaft-Stumpfmodells (109; 401; 500; 600) zur Herstellung eines Prothesenschaftes zur Verbindung eines einen Stumpf bildenden Körperteils mit einer Prothese, die Schritte umfassend:
a) Akquirieren von dreidimensionalen Bilddaten (101) des den Stumpf bildenden Körperteils, das mehrere Gewebearten umfasst;
b) Segmentieren (103; 808) der 3D-Bilddaten (103) zur Bestimmung der Verteilung von wenigstens einer Gewebeart des Stumpfs;
c) Rekonstruieren (108; 812) eines 3D-Schaft-Stumpfmodells (109; 500; 600) anhand der segmentierten 3D-Bilddaten, das die Geometrie des Stumpfes und Verteilung der wenigstens einen segmentierten Gewebeart des Stumpfes beschreibt;
d) Festlegen (816) anhand des 3D-Schaft-Stumpfmodells (109; 500; 600) wenigstens einer Stumpfachse (207; 614; 1204); und
e) Unterteilen wenigstens eines Bereiches des 3D-Schaft-Stumpfmodells (109; 401) in wenigstens eine Scheibe (301; 700; 900; 1106) einer bestimmten Dicke senkrecht zur Stumpfachse (207; 614; 1204); **gekennzeichnet durch**
f) Unterteilen der wenigstens einen Scheibe (301; 700; 900; 1106) in Winkelsektoren (302; 702; 1312);
g) Modifizieren des 3D-Schaft-Stumpfmodells (110; 828) anhand von wissensbasierten Regelsätzen, die auf wenigstens einen Winkelsektor (302; 702; 1312) der wenigstens einen Scheibe (301; 700; 900; 1106) angewendet werden, zur optimalen Anpassung des 3D-Schaft-Stumpfmodells an den Stumpf, wobei die wissensbasierten Regelsätze die in dem 3D-Schaft-Stumpfmodell (109; 500; 600) enthaltenen Informationen über Geometrie des Stumpfes und/oder Verteilung der wenigstens einen segmentierten Gewebeart berücksichtigen, und
eine oder mehrere Regeln umfassen, die eine oder mehrere Eigenschaften der wenigstens einen segmentierten Gewebeart verwenden.

16. Verfahren nach Anspruch 15, wobei die Dicke der wenigstens eine Scheibe (301; 700; 900; 1106) in einem proximalen (608) Bereich größer bestimmt wird als in einem distalen (616) Bereich.

17. Verfahren nach Anspruch 15 oder 16, wobei das Unterteilen der wenigstens einen Scheibe (301; 700; 900; 1106) ein Unterteilen in Winkelsektoren (302; 702; 1312) umfasst, welche medial (716) kleinere Winkelanteile aufweisen als lateral (714).

18. Verfahren nach einem der Ansprüche 15 bis 17, wobei das Unterteilen (824) der wenigstens einen Scheibe (301; 700; 900; 1106) in Winkelsektoren (302; 702; 1312) basierend auf der Auswahl wenigstens einer vorab festgelegten Winkelsektor-Unterteilung durch einen Benutzer (1102) durchgeführt wird.

19. Verfahren nach Anspruch 18, wobei die vorab festgelegten Winkelsektor-Unterteilungen, aus denen der Benutzer (1102) für die Scheibe (301; 700; 900; 1106) auswählen kann, abhängen von:
- der Lage der Scheibe (301; 700; 900; 1106) innerhalb des Stumpfes;
- der Gewebeverteilung in der Scheibe (301; 700; 900; 1106); und/oder
physiologischen und anatomischen Eigenschaften des Stumpfes bzw. des Patienten.

20. Verfahren nach Anspruch 18 oder 19, wobei die vorab festgelegten Winkelsektor-Unterteilungen, aus denen der Benutzer (1102) für die Scheibe (301; 700; 900; 1106) auswählen kann, eine physiologisch zu erwartende Veränderung des Stumpfes berücksichtigen.

21. Verfahren nach einem der Ansprüche 18 bis 20, wobei der Satz vorab festgelegter Winkelsektor-Unterteilungen basierend auf der Analyse von voraus gegangenen Modifizierungen des 3D-Schaft-Stumpfmodells anderer und/oder desselben Patienten angepasst wird.

22. Verfahren nach einem der Ansprüche 18 bis 21, wobei die ausgewählte Winkelsektor-Unterteilung durch den Benutzer (1102) um die Stumpfachse (207; 614; 1204) gedreht (709; 826) werden kann.

23. Verfahren nach einem der Ansprüche 15 bis 22, wobei das Modifizieren eine Volumenkompression umfasst, wobei die wissensbasierten Regelsätze wenigstens einen Faktor zur Volumenkompression umfassen, der ein prozentualer Kompressionswert für eine der im Winkelsektor (302; 702; 1312) enthaltenen segmentierten Gewebearten (104-107; 704-707) ist.

24. Verfahren nach Anspruch 23, bei dem der Faktor manuell durch den Benutzer (1102) verändert werden kann.

25. Verfahren nach einem der Ansprüche 15 bis 24, bei dem die Gewebearten Haut (104; 704), Fett (105; 705), Muskeln (106; 706) und Knochen (107; 707) umfassen.

26. Verfahren nach einem der Ansprüche 15 bis 25, bei dem während dem Akquirieren der 3D-Bilddaten (101) des den Stumpf bildenden Körperteils ein Liner (206) zur Formung des Stumpfes angebracht ist, wobei das Material des Liners (206) derart gewählt wird, dass es sich zur Segmentierung (103; 808) in den 3D-Bilddaten eignet.

27. Verfahren nach einem der Ansprüche 15 bis 26, wobei das Segmentieren (103; 808) der 3D-Bilddaten anhand von 2D-Darstellungen der 3D-Bilddaten durchgeführt wird.

28. Verfahren nach einem der Ansprüche 15 bis 27, bei dem die wissensbasierten Regelsätze medizinische Erfahrungswerte in Form von wenigstens einer mathematischen Transformationsvorschrift zum Modifizieren (110; 828) des 3D-Schaft-Stumpfmodells (109; 500; 600) umfassen.

29. Verfahren nach einem der Ansprüche 15 bis 28, bei dem das Modifizieren (110; 828) des 3D-Schaft-Stumpfmodells (109; 500; 600) anhand von wissensbasierten Regelsätzen ein Modifizieren (110; 828) auf der Grundlage der äußeren Form, bevorzugt deren Krümmungen, des 3D-Schaft-Stumpfmodells (109; 500; 600) und/oder ein Modifizieren (110; 828) zur optimalen Anpassung des 3D-Schaft-Stumpfmodells (109; 500; 600) an wesentliche Knochenstrukturen (203, 205) umfasst.

30. Verfahren nach einem der Ansprüche 15 bis 29, wobei das Modifizieren anhand wissensbasierter Regelsätze des 3D-Schaft-Stumpf-Modells (190; 401; 500; 600) eine Volumenänderung berücksichtigt, welche im Wesentlichen parallel zur Stumpfachse (207; 614; 1204) verläuft.

31. Verfahren nach einem der Ansprüche 15 bis 30, wobei beim Modifizieren eine Oberflächenglättung des modifizierten 3D-Schaft-Stumpfmodells (401) durchgeführt wird, wobei die Oberflächenglättung (806; 830) zwischen zwei benachbarten Scheiben (301; 700; 900; 1106) erfolgt.

32. Computerprogramm, welches angepasst ist, um das Verfahren nach einem der Ansprüche 15 bis 31 durchzuführen.

## Claims

1. An apparatus for the interaction (1110) of a user (1102) with a 3D shaft / stump computer model in order to modify the 3D shaft / stump computer model (109; 401; 500; 600) which describes the surface shape and spatial tissue distribution of a stump, wherein the apparatus is designed to subdivide the 3D socket / stump computer model (109; 401; 500; 600) into portions (301; 700; 900; 1106), with
- a display (1100) which is designed to display the surface shape and tissue distribution in a portion (301; 700; 900; 1106) of the 3D socket / stump computer model (109; 401; 500; 600),
- a first selection module which allows the user (1102) to select (1108) a portion (301; 700; 900; 1106) of the 3D socket / stump computer model (109; 401; 500; 600) for display on the display (1100), **characterized by**
- a second selection module (1112) which allows the user (1102) to select at least one previously set spatial distribution of a modifiability of the surface shape in the portion (301; 700; 900; 1106), wherein the apparatus is designed to modify (110; 828; 1116) the surface shape in the portion (301; 700; 900; 1106) according to the selected spatial distribution.

2. An apparatus according to claim 1, wherein the apparatus is designed to allow the user (1102) to set a stump axis (207; 614; 1204) with reference to the 3D socket / stump computer model (109; 401; 500; 600), and is designed to subdivide the 3D socket / stump computer model (109; 401; 500; 600) into portions (301; 700; 900; 1106) which are orientated on the stump axis (207; 614; 1204).

3. An apparatus according to claim 2, wherein the portions (301; 700; 900; 1106) are discs (301; 700; 900; 1106) situated substantially at a right angle to the stump axis (207; 614; 1204), and the apparatus is designed to allow the user (1102) to determine the thickness of a disc (301; 700; 900; 1106) individually.

4. An apparatus according to any one of the preceding claims, wherein the spatial distribution set beforehand comprises a subdivision of the portion (301; 700; 900; 1106) in at least one subsidiary area (302; 702; 1312), wherein each subsidiary area (302; 702; 1312) has associated with it at least one value (1314) on which the degree of the modification of the surface shape is based.

5. An apparatus according to claim 4, which is designed so that on the basis of the value (1314) and the tissue distribution in the subsidiary area (302; 702; 1312) it derives a change in volume of one of the types of tissue contained the subsidiary area (302; 702; 1312) and determines the degree of the modification of the surface shape with reference to the change in volume.

6. An apparatus according to claim 5, wherein the types of tissue comprise fat, muscle, skin and/or bone and the value (1314) indicates the percentage change in volume of the respective type of tissue (104 - 107; 704 - 707) and is based upon the compressibility of the respective type of tissue.

7. An apparatus according to claims 4 to 6, in which the second selection module (1112) allows the user (1102) to change the value (1314) manually.

8. An apparatus according to claims 4 to 6, wherein the subsidiary area (302; 702; 1312) is an angular sector (302; 702; 1312) which is arranged radially starting from the stump axis (207; 614; 1204).

9. An apparatus according to claim 7 or 8, in which the second selection module (1112) allows the user (1102) to change the spatial orientation of the angular sector (302; 702; 1312) manually by rotation (709) about the stump axis (207; 614; 1204).

10. An apparatus according to any one of the preceding claims, designed in such a way that with the modification (110; 828; 1116) the shape of the face of the subsidiary area (302; 702; 1312) forming the outer face of the 3D socket / stump computer model (109; 401; 500; 600) is essentially retained.

11. An apparatus according to any one of the preceding claims, in which the second selection module (1112) is designed in such a way that the spatial distributions which are set beforehand and from which the user (1102) can choose for the respective portion (301; 700; 900; 1106) depend upon:
- the position of the portion (301; 700; 900; 1106) inside the stump;
- the tissue distribution of the portion (301; 700; 900; 1106) and/or
- physiological or anatomical properties of the stump or of the patent respectively.

12. An apparatus according to any one of the preceding claims, which also comprises a data base module in which the spatial distributions set beforehand are filed, and the apparatus is designed, on the basis of an analysis of previous modifications (110; 828; 1116) of the 3D socket / stump computer model (109; 401; 500; 600) of other patients and/or the same patient, to adapt the set of the spatial distributions in the data base.

13. An apparatus according to any one of the preceding claims, in which the second selection module (1112) is designed in such a way that the spatial distributions which are set beforehand and from which the user (1102) can choose for the respective portion (301; 700; 900; 1106) take into consideration a change in the stump which is be expected physiologically.

14. A system of interaction (1110) of a user (1102) with a 3D socket / stump computer model (109; 401; 500; 600) in order to modify the 3D shaft / stump computer model (109; 401; 500; 600), wherein the system comprises:
a) a read-in unit for reading in 3D image data of the stump;
b) a segmentation unit (103) for segmenting the 3D image data in order to determine the spatial tissue distribution of the stump;
c) a reconstruction unit (108) for reconstructing a 3D socket / stump computer model (109; 401; 500; 600) with reference to the segmented 3D image data, which describes the surface shape and the spatial tissue distribution of the stump;
d) an apparatus for interacting (1110) with a user (1102) according to any one of claims 1 to 13, which modifies the 3D socket / stump computer model (109; 401; 500; 600) whilst taking into consideration inputs by the user (1102), and
e) an output unit which outputs the modified 3D shaft / stump computer model (109; 401; 500; 600) for further use for the production of a prosthesis socket.

15. A method of creating a 3D socket / stump model (109; 401; 500; 600) in order to produce a prosthesis socket for connecting a part of the body forming a stump to a prosthesis, comprising the steps:
a) acquiring three-dimensional image data (101) of the part of the body which forms the stump and which comprises a multiplicity of types of tissue;
b) segmentation (103; 808) of the 3D image data (103) in order to determine the distribution of at least one type of tissue of the stump;
c) reconstruction (108; 812) of a 3D socket / stump model (109; 500; 600) with reference to the segmented 3D image data, which describes the geometry of the stump and the distribution of the at least one segmented type of tissue of the stump;
d) establishing (816) at least one stump axis (207; 614; 1204) with reference to the 3D socket / stump model (109; 500; 600), and
e) subdivision of at least one area of the 3D socket / stump model (109; 401) into at least one disc (301; 700; 900; 1106) of a pre-determined thickness at a right angle to the stump axis (207; 614; 1204), **characterized by**
f) subdivision of the at least one disc (301; 700; 900; 1106) into angular sectors (302; 702; 1312),
g) modification of the 3D socket / stump model (110; 828) with reference to knowledge-based sets of rules which are applied to at least one angular sector (302; 702; 1312) of the at least one disc (301; 700; 900; 1106), for the optimum adaptation of the 3D socket / stump model to the stump, wherein the knowledge-based sets of rules
take into consideration the information contained in the 3D socket / stump model (109; 500; 600) on the geometry of the stump and/or the distribution of the at least one segmented type of tissue, and
comprise one or more rules which apply one or more properties of the at least one segmented type of tissue.

16. A method according to claim 15, wherein the thickness of the at least one disc (301; 700; 900; 1106) is set to be greater in a proximal (608) area than in a distal (616) area.

17. A method according to claim 15 or 16, wherein the subdivision of the at least one disc (301; 700; 900; 1106) comprises a subdivision into angular sectors (302; 702; 1312) which have smaller angular portions medially (716) than laterally (714).

18. A method according to any one of claims 15 to 17, wherein the subdivision (824) of the at least one disc (301; 700; 900; 1106) into angular sectors (302; 702; 1312) is carried out by a user (1102) on the basis of the choice of at least one angular sector subdivision set beforehand.

19. A method according to claim 18, wherein the angular sector subdivisions which are set beforehand and from which the user (1102) can choose for the disc (301; 700; 900; 1106) depend upon:
- the position of the disc (301; 700; 900; 1106) inside the stump;
- the tissue distribution in the disc (301; 700; 900; 1106), and/or
- physiological and anatomical properties of the stump or of the patent respectively.

20. A method according to claim 18 or 19, wherein the angular sector subdivisions which are set beforehand and from which the user (1102) can choose for the disc (301; 700; 900; 1106) take into consideration a change in the stump which is be expected physiologically.

21. A method according to any one of claims 18 to 20, wherein the set of angular sector subdivisions set beforehand are adapted on the basis of the analysis of previously performed modifications of the 3D socket / stump model of other patients and/or the same patient.

22. A method according to any one of claims 18 to 21, wherein the angular sector subdivision chosen can be rotated (709; 826) by the user (1102) about the stump axis (207; 614; 1204).

23. A method according to any one of claims 15 to 22, wherein the modification comprises a volume compression, wherein the knowledge-based sets of rules comprise at least one factor for the volume compression which is a percentage compression value for one of the segmented types of tissue (104 - 107; 704 - 707) contained in the angular sector (302; 702; 1312).

24. A method according to claim 23, in which the factor can be changed manually by the user (1102).

25. A method according to any one of claims 15 to 24, in which the types of tissue comprise skin (104; 704), fat (105; 705), muscles (106; 706) and bone (107; 707).

26. A method according to any one of claims 15 to 25, in which during the acquisition of the 3D image data (101) of the part of the body forming the stump a liner (206) for forming the stump is attached, wherein the material of the liner (206) is chosen in such a way that it is suitable for segmentation (103; 808) in the 3D image data.

27. A method according to any one of claims 15 to 26, wherein the segmentation (103; 808) of the 3D image data is carried out with reference to 2D representations of the 3D image data.

28. A method according to any one of claims 15 to 27, in which the knowledge-based sets of rules comprise medicinal empirical values in the form of at least one mathematical transformation instruction for the modification (110; 828) of the 3D socket / stump model (109; 500; 600).

29. A method according to any one of claims 15 to 28, in which the modification (110; 828) of the 3D socket / stump model (109; 500; 600) with reference to knowledge-based sets of rules comprises a modification (110; 828) on the basis of the outer shape, preferably its curves, of the 3D socket / stump model (109; 500; 600) and/or a modification (110; 828) for the optimum adaptation of the 3D socket / stump model (109; 500; 600) to essential bone structures (203, 205).

30. A method according to any one of claims 15 to 29, wherein the modification with reference to knowledge-based sets of rules of the 3D socket / stump model (190 [*sic*]; 401; 500; 600) takes into consideration a change in volume which extends substantially parallel to the stump axis (207; 614; 1204).

31. A method according to any one of claims 15 to 30, wherein a surface smoothing of the modified 3D socket / stump model (401) is carried out in the modification, wherein the surface smoothing is carried out between two adjacent discs (301; 700; 900; 1106).

32. A computer program which is adapted to carry out the method according to any one of claims 15 to 31.

## Revendications

1. Dispositif pour l'interaction (1110) d'un utilisateur (1102) avec un modèle informatique d'emboîture de moignon en 3D afin de modifier le modèle informatique d'emboîture de moignon en 3D (109 ; 401 ; 500 ; 600) qui décrit la forme superficielle et la répartition des tissus d'un moignon dans l'espace, le dispositif étant configuré pour diviser le modèle informatique d'emboîture de moignon en 3D (109 ; 401 ; 500 ; 600) en sections (301 ; 700 ; 900 ; 1106), ledit dispositif comportant :
- un écran (1100) qui est conçu pour afficher la forme superficielle et la répartition des tissus dans une section (301 ; 700 ; 900 ; 1106) du modèle informatique d'emboîture de moignon en 3D (109 ; 401 ; 500 ; 600) ; et
- un premier module de sélection permettant à l'utilisateur (1102) de sélectionner (1108) une section (301 ; 700 ; 900 ; 1106) du modèle informatique d'emboîture de moignon en 3D (109 ; 401 ; 500 ; 600) pour l'afficher (1108) sur l'écran (1100) ;
**caractérisé par**
- un deuxième module de sélection (1112) permettant à l'utilisateur (1102) de sélectionner au moins une répartition dans l'espace, définie au préalable, d'une possibilité de modification de la forme superficielle dans la section (301 ; 700 ; 900 ; 1106) ;
le dispositif étant configuré pour modifier (110 ; 828 ; 1116) la forme superficielle dans la section (301 ; 700 ; 900 ; 1106) conformément à la répartition dans l'espace sélectionnée.

2. Dispositif selon la revendication 1, dans lequel le dispositif est configuré pour permettre à l'utilisateur (1102) de définir un axe de moignon (207 ; 614 ; 1204) à l'appui du modèle informatique d'emboîture de moignon en 3D (109 ; 401 ; 500 ; 600), et est configuré pour diviser le modèle informatique d'emboîture de moignon en 3D (109 ; 401 ; 500 ; 600) en sections (301 ; 700 ; 900 ; 1106) qui sont alignés sur l'axe de moignon (207 ; 614 ; 1204).

3. Dispositif selon la revendication 2, dans lequel les sections (301 ; 700 ; 900 ; 1106) sont des disques (301 ; 700 ; 900 ; 1106) sensiblement perpendiculaires à l'axe de moignon (207 ; 614 ; 1204), et le dispositif est configuré pour permettre à l'utilisateur (1102) de déterminer individuellement l'épaisseur d'un disque (301 ; 700 ; 900 ; 1106).

4. Dispositif selon l'une quelconque des revendications précédentes, dans lequel la répartition dans l'espace, définie au préalable, comprend une subdivision de la section (301 ; 700 ; 900 ; 1106) en au moins une zone partielle (302 ; 702 ; 1312), à chaque zone partielle (302 ; 702 ; 1312) étant associée au moins une valeur (1314), sur laquelle est basée l'importance de la modification de la forme superficielle.

5. Dispositif selon la revendication 4, qui est configuré de telle sorte que, sur la base de la valeur (1314) et de la répartition des tissus dans la zone partielle (302 ; 702 ; 1312), il déduit une variation de volume d'un des types de tissu contenus dans la zone partielle (302 ; 702 ; 1312) et, à l'appui de la variation de volume, il détermine l'importance de la modification de la forme superficielle.

6. Dispositif selon la revendication 5, dans lequel les types de tissu comprennent la graisse, les muscles, la peau et/ou les os, et la valeur (1314) indique la variation proportionnelle du volume du type de tissu (104-107 ; 704-707) respectif et est basée sur la compressibilité du type de tissu respectif.

7. Dispositif selon l'une quelconque des revendications 4 à 6, dans lequel le deuxième module de sélection (1112) permet à l'utilisateur (1102) de modifier manuellement la valeur (1314).

8. Dispositif selon l'une quelconque des revendications 4 à 6, dans lequel la zone partielle (302 ; 702 ; 1312) est un secteur angulaire (302 ; 702 ; 1312) qui est agencé radialement en partant de l'axe de moignon (207 ; 614 ; 1204).

9. Dispositif selon la revendication 7 ou 8, dans lequel le deuxième module de sélection (1112) permet à l'utilisateur (1102) de modifier manuellement l'orientation dans l'espace du secteur angulaire (302 ; 702 ; 1312) par rotation (709) autour de l'axe de moignon (207 ; 614 ; 1204).

10. Dispositif selon l'une quelconque des revendications précédentes, qui est configuré de telle sorte que la forme de la surface de la zone partielle (302 ; 702 ; 1312), formant la face extérieure du modèle informatique d'emboîture de moignon en 3D (109 ; 401 ; 500 ; 600), est conservée pour l'essentiel pendant la modification (110 ; 828 ; 1116).

11. Dispositif selon l'une quelconque des revendications précédentes, dans lequel le deuxième module de sélection (1112) est configuré de telle sorte que les répartitions dans l'espace définies au préalable, parmi lesquelles l'utilisateur (1102) peut sélectionner la section (301 ; 700 ; 900 ; 1106) respective, dépendent :
- de la position de la section (301 ; 700 ; 900 ; 1106) à l'intérieur du moignon ;
- de la répartition des tissus de la section (301 ; 700 ; 900 ; 1106) ; et/ou
- des propriétés physiologiques ou anatomiques du moignon ou du patient.

12. Dispositif selon l'une quelconque des revendications précédentes, qui comprend également un module pour base de données, dans lequel sont stockées les répartitions dans l'espace définies au préalable, et le dispositif est configuré pour ajuster l'enregistrement des répartitions dans l'espace dans la base de données en se basant sur une analyse des modifications (110 ; 828 ; 1116) préalables du modèle informatique d'emboîture de moignon en 3D (109 ; 401 ; 500 ; 600) d'autres patients et/ou du même patient.

13. Dispositif selon l'une quelconque des revendications précédentes, dans lequel le deuxième module de sélection (1112) est configuré de telle sorte que les répartitions dans l'espace définies au préalable parmi lesquelles l'utilisateur (1102) peut sélectionner la section (301 ; 700 ; 900 ; 1106) respective, tiennent compte d'une variation du moignon à escompter sur le plan physiologique.

14. Système pour l'interaction (1110) d'un utilisateur (1102) avec un modèle informatique d'emboîture de moignon en 3D (109 ; 401 ; 500 ; 600) afin de modifier le modèle informatique d'emboîture de moignon en 3D (109 ; 401 ; 500 ; 600), ledit système comprenant :
a) une unité de saisie pour entrer des données d'images en 3D du moignon ;
b) une unité de segmentation (103) pour segmenter les données d'images en 3D afin de déterminer la répartition dans l'espace des tissus du moignon ;
c) une unité de reconstruction (108) pour reconstruire un modèle informatique d'emboîture de moignon en 3D (109 ; 401 ; 500 ; 600) à l'appui des données d'images en 3D segmentées, lequel décrit la forme superficielle et la répartition dans l'espace des tissus du moignon ;
d) un dispositif pour l'interaction (1110) avec un utilisateur (1102) selon l'une quelconque des revendications 1 à 13, lequel modifie le modèle informatique d'emboîture de moignon en 3D (109 ; 401 ; 500 ; 600) en tenant compte des entrées de l'utilisateur (1102) ; et
e) une unité d'édition qui édite le modèle informatique d'emboîture de moignon en 3D (109 ; 401 ; 500 ; 600) modifié afin de l'utiliser ultérieurement pour la fabrication d'une emboîture de prothèse.

15. Procédé pour la création d'un modèle d'emboîture de moignon en 3D (109 ; 401 ; 500 ; 600) pour la fabrication d'une emboîture de prothèse, destinée à relier une partie d'un corps en forme de moignon à une prothèse, ledit procédé comportant les étapes :
a) acquisition de données d'images tridimensionnelles (101) de la partie du corps en forme de moignon comprenant plusieurs types de tissus ;
b) segmentation (103 ; 808) des données d'images en 3D (103) pour déterminer la répartition dudit au moins un type de tissu du moignon ;
c) reconstruction (108 ; 812) d'un modèle d'emboîture de moignon en 3D (109 ; 500 ; 600) à l'appui des données d'images en 3D segmentées, lequel décrit la géométrie du moignon et la répartition dudit au moins un type de tissu segmenté du moignon ;
d) définition (816) à l'appui du modèle d'emboîture de moignon en 3D (109 ; 500 ; 600) d'au moins un axe de moignon (207 ; 614 ; 1204) ; et
e) subdivision d'au moins une zone du modèle d'emboîture de moignon en 3D (109 ; 401) en au moins un disque (301 ; 700 ; 900 ; 1106) d'une épaisseur déterminée perpendiculairement à l'axe de moignon (207 ; 614 ; 1204) ;
caractérisé les étapes de
f) subdivision dudit au moins un disque (301 ; 700 ; 900 ; 1106) en secteurs angulaires (302 ; 702 ; 1312) ;
g) modification du modèle d'emboîture de moignon en 3D (110 ; 828) à l'appui des ensembles de règles basés sur les connaissances, qui sont appliqués à au moins un secteur angulaire (302 ; 702 ; 1312) dudit au moins un disque (301 ; 700 ; 900 ; 1106), pour un ajustement optimal du modèle d'emboîture de moignon en 3D au moignon, les ensembles de règles basés sur les connaissances
tenant compte des informations sur la géométrie du moignon et/ou sur la répartition dudit au moins un type de tissu segmenté, contenues dans le modèle d'emboîture de moignon en 3D (109 ; 500 ; 600), et
comprenant une ou plusieurs règles qui utilisent une ou plusieurs propriétés dudit au moins un type de tissu segmenté.

16. Procédé selon la revendication 15, selon lequel l'épaisseur dudit au moins un disque (301 ; 700 ; 900 ; 1106) dans une zone proximale (608) est déterminée pour être supérieure à celle dans une zone distale (616).

17. Procédé selon la revendication 15 ou 16, selon lequel la subdivision dudit au moins un disque (301 ; 700 ; 900 ; 1106) comprend une subdivision en secteurs angulaires (302 ; 702 ; 1312) qui ont des valeurs angulaires plus petites en médial (716) qu'en latéral (714).

18. Procédé selon l'une quelconque des revendications 15 à 17, selon lequel la subdivision (824) dudit au moins un disque (301 ; 700 ; 900 ; 1106) en secteurs angulaires (302 ; 702 ; 1312) est effectuée par un utilisateur (1102) sur la base de la sélection d'au moins une subdivision en secteurs angulaires définie au préalable.

19. Procédé selon la revendication 18, selon lequel les subdivisions en secteurs angulaires définies au préalable parmi lesquelles l'utilisateur (1102) peut sélectionner le disque (301 ; 700 ; 900 ; 1106) dépendent
- de la position de la section (301 ; 700 ; 900 ; 1106) à l'intérieur du moignon ;
- de la répartition des tissus dans le disque (301 ; 700 ; 900 ; 1106) ; et/ou
- des propriétés physiologiques ou anatomiques du moignon ou du patient.

20. Procédé selon la revendication 18 ou 19, selon lequel les subdivisions en secteurs angulaires définies au préalable parmi lesquelles l'utilisateur (1102) peut sélectionner le disque (301 ; 700 ; 900 ; 1106) tiennent compte d'une variation du moignon à escompter sur le plan physiologique.

21. Procédé selon l'une quelconque des revendications 18 à 20, selon lequel l'enregistrement des subdivisions en secteurs angulaires définies au préalable est ajusté sur la base d'une analyse des modifications préalables du modèle d'emboîture de moignon en 3D d'autres patients et/ou du même patient.

22. Procédé selon l'une quelconque des revendications 18 à 21, selon lequel l'utilisateur (1102) peut faire tourner (709 ; 826) autour de l'axe de moignon (207 ; 614 ; 1204) la subdivision en secteurs angulaires sélectionnée.

23. Procédé selon l'une quelconque des revendications 15 à 22, selon lequel la modification comprend une compression du volume, les ensembles de règles basés sur les connaissances comprenant au moins un facteur de compression du volume qui est une valeur de compression proportionnelle pour un des types de tissu (104-107 ; 704-707) segmentés contenus dans le secteur angulaire (302 ; 702 ; 1312).

24. Procédé selon la revendication 23, selon lequel le facteur peut être modifié manuellement par l'utilisateur (1102).

25. Procédé selon l'une quelconque des revendications 15 à 24, selon lequel les types de tissu comprennent la peau (104, 704), la graisse (105 ; 705), les muscles (106 ; 706) et les os (107 ; 707).

26. Procédé selon l'une quelconque des revendications 15 à 25, selon lequel, pendant l'acquisition de données d'images en 3D (101) de la partie du corps en forme de moignon, un film (206) est fourni pour le formage du moignon, le matériau du film (206) étant choisi de telle sorte qu'il convient à la segmentation (103 ; 808) en données d'images en 3D.

27. Procédé selon l'une quelconque des revendications 15 à 26, selon lequel la segmentation (103 ; 808) des données d'images en 3D est effectuée à l'appui de représentations en 2D des données d'images en 3D.

28. Procédé selon l'une quelconque des revendications 15 à 27, selon lequel les ensembles de règles basés sur les connaissances comprennent des valeurs d'expérience médicale sous la forme d'au moins une règle de transformation mathématique pour la modification (110 ; 828) du modèle d'emboîture de moignon en 3D (109 ; 500 ; 600).

29. Procédé selon l'une quelconque des revendications 15 à 28, selon lequel la modification (110 ; 828) du modèle d'emboîture de moignon en 3D (109 ; 500 ; 600) à l'appui des ensembles de règles basés sur les connaissances comprend une modification (110 ; 828) sur la base de la forme extérieure, de préférence les courbures de celle-ci, du modèle d'emboîture de moignon en 3D (109 ; 500 ; 600) et/ou une modification (110 ; 828) pour l'ajustement optimal du modèle d'emboîture de moignon en 3D (109 ; 500 ; 600) aux principales structures osseuses (203, 205).

30. Procédé selon l'une quelconque des revendications 15 à 29, selon lequel la modification à l'appui des ensembles de règles basés sur les connaissances du modèle d'emboîture de moignon en 3D (109 ; 500 ; 600) tient compte d'une modification du volume qui s'étend sensiblement parallèlement à l'axe de moignon (207 ; 614 ; 1204).

31. Procédé selon l'une quelconque des revendications 15 à 30, selon lequel un lissage de surface du modèle d'emboîture de moignon en 3D (401) modifié est effectué pendant la modification, ledit lissage de surface (806 ; 803) s'effectuant entre deux disques (301 ; 700 ; 900 ; 1106) adjacents.

32. Programme informatique qui est adapté à la mise en ouvre du procédé selon l'une quelconque des revendications 15 à 31.
